# EUROPEAN PATENT SPECIFICATION

(11) **EP 4 346 816 B1**
(45) Date of publication and mention of the grant of the patent: **30.09.2026**
(21) Application number: 22733550.2
(22) Date of filing: 31.05.2022
(51) Int. Cl.: A61K 31/44, A61K 31/4412, A61K 31/351, A61P 17/06, A61P 19/02, A61P 21/04, A61P 29/00, A61P 37/00

(54) **COMPOUNDS FOR USE IN A METHOD FOR TREATING AN AUTOIMMUNE AND INFLAMMATORY DISEASE**
VERBINDUNGEN ZUR VERWENDUNG IN EINEM VERFAHREN ZUR BEHANDLUNG EINER AUTOIMMUN- UND ENTZÜNDUNGSERKRANKUNG
COMPOSÉS POUR UNE UTILISATION DANS UN PROCÉDÉ DE TRAITEMENT DE MALADIES AUTO-IMMUNES ET INFLAMMATOIRES

(30) Priority: 02.06.2021 EP 21177351
(43) Date of publication of application: 10.04.2024
(73) Proprietor: Cellestia Biotech AG, 4057 Basel (CH)
(72) Inventor: LEHAL, Rajwinder, 8004 Zürich (CH); VIGOLO, Michele, 1007 Lausanne (CH); MAGNIN, Morgane, 39400 Morez (FR); LAMY, Sebastien, 01170 Echenevex (FR)
(74) Representative: Latscha Schöllhorn Partner AG
(86) International application number: PCT/EP2022/064684
(87) International publication number: WO 2022/253794

(56) References cited:
- WO-A1-2020/208139
- WO-A1-2020/209933
- WO-A2-2004/087195
- KANG JIN HYUN ET AL: "Regulation of Th1 and Th2 phenotypes by gamma-secretase inhibitor in an animal model for asthma", JOURNAL OF IMMUNOLOGY, vol. 182, no. Suppl. 1, 1 April 2009 (2009-04-01), & 96TH ANNUAL MEETING OF THE AMERICAN-ASSOCIATION-OF-IMMUNOLOGISTS (AAI); SEATTLE, WA, USA; MAY 08 -12, 2009, pages 79.10, XP002804763, ISSN: 0022-1767
- ZHOU MIN ET AL: "Blockade of Notch Signalling by [gamma]-Secretase Inhibitor in Lung T Cells of Asthmatic Mice Affects T Cell Differentiation and Pulmonary Inflamma", INFLAMMATION, PLENUM PRESS, NEW YORK, NY, US, vol. 38, no. 3, 15 January 2015 (2015-01-15), pages 1281 - 1288, XP035496570, ISSN: 0360-3997, [retrieved on 20150115], DOI: 10.1007/S10753-014-0098-5
- MINTER LISA M ET AL: "Inhibitors of gamma-secretase block in vivo and in vitro T helper type 1 polarization by preventing Notch upregulation of Tbx21", NATURE IMMUNOLOGY, vol. 6, no. 7, July 2005 (2005-07-01), pages 680 - 688, XP002804764, ISSN: 1529-2908
- MA LEI ET AL: "Effect of [gamma]-secretase inhibitor on Th17 cell differentiation and function of mouse psoriasis-like skin inflammation.", JOURNAL OF TRANSLATIONAL MEDICINE 10 03 2018, vol. 16, no. 1, 10 March 2018 (2018-03-10), pages 59, XP002804765, ISSN: 1479-5876
- JIAO Z J ET AL: "Modulating Notch pathway using @c-secretase inhibitors for the immunotherapy of rheumatoid arthritis", BIOSCIENCE HYPOTHESES,, vol. 1, no. 6, 1 January 2008 (2008-01-01), pages 332 - 333, XP025714096, ISSN: 1756-2392, [retrieved on 20081105], DOI: 10.1016/J.BIHY.2008.08.008
- LEHAL RAJWINDER ET AL: "Pharmacological disruption of the Notch transcription factor complex", PROCEEDINGS OF THE NATIONAL ACADEMY OF SCIENCES, vol. 117, no. 28, 29 June 2020 (2020-06-29), pages 16292 - 16301, XP055803866, ISSN: 0027-8424, DOI: 10.1073/pnas.1922606117
- LEHAL R ET AL: "PHARMACOLOGICAL ACTIVITY OF CB-103-AN ORAL PAN-NOTCH INHIBITOR WITH A NOVEL MODE OF ACTION", HAEMATOLOGICA, FONDAZIONE FERRATA STORTI, IT, vol. 102, no. Suppl. 2, 26 June 2017 (2017-06-26), pages 29, XP002805694, ISSN: 0390-6078
- KLEINJAN ALEX ET AL: "The Notch pathway inhibitor stapled alpha-helical peptide derived from mastermind-like 1 (SAHM1) abrogates the hallmarks of allergic asthma", JOURNAL OF ALLERGY AND CLINICAL IMMUNOLOGY, vol. 142, no. 1, July 2018 (2018-07-01), pages 76 - 85, XP002805695, ISSN: 0091-6749
- L. ASTUDILLO ET AL: "The Small Molecule IMR-1 Inhibits the Notch Transcriptional Activation Complex to Suppress Tumorigenesis", CANCER RESEARCH, vol. 76, no. 12, 15 June 2016 (2016-06-15), US, pages 3593 - 3603, XP055320374, ISSN: 0008-5472, DOI: 10.1158/0008-5472.CAN-16-0061

## Description

### Field of the invention

The present invention is defined in the appended claims. It relates to inhibitors of Notch transcription activation complex of formula (I) below for use in methods for the prevention, delay of progression or treatment of an autoimmune and inflammatory disease (AIID) in a subject.

### Background of the invention

Autoimmune and inflammatory diseases (AIIDs) encompass >150 diseases, each resulting from a host immune response against self or non-self antigens, and affect up to 50 million individuals in the U.S. alone. AIIDs can be broadly characterized into five sub-categories based on type of immune response (e.g., adaptive vs. innate) and origin of the antigen (e.g., self vs. non-self) the host immune system is reacting to. The five sub-categories are inflammatory diseases; autoimmune diseases; allergic diseases; autoinflammatory diseases; and Graft-versus-Host-Disease (GvHD)/transplant rejection. New treatments targeting AIIDs are being investigating e.g., NLRP3/inflammasome inhibitors and antigen-specific immune tolerizing approaches, in order to meet the high medical need. WO 2004/087195, WO 2020/209933 and KleinJan A. et al. (Journal of Allergy and Clinical Immunology (2017), 142(1), 76 - 85) disclose Notch pathway modulators and their use for the treatment of autoimmune inflammatory diseases.

### Summary of the invention

It has now unexpectedly been found that T cell modulators are useful for the prevention, delay of progression or treatment of an autoimmune and inflammatory disease (AIID). Surprisingly, it has been found that the effect of T cell modulators in prevention, delay or treatment of AIID can be mediated via direct action on conventional T cells or immune suppressive regulatory T cells (Treg cells). T cell modulators such as 6-(4-tert-butylphenoxy)pyridin-3-amine led to expansion of Treg cells in human and mouse PBMC culture stimulation and treatment experiments. In addition, T cell modulators resulted in a significant lower cumulative GvHD score, prevented GvHD andenhanced GvHD free survival *in vivo* compared to the control. Taking these unexpected findings into account, the inventors herewith provide the present invention in its following aspects.

In a first aspect, the present invention provides a T cell modulator (TCM) for use in a method for the prevention, delay of progression or treatment of an autoimmune and inflammatory disease (AIID) in a subject, wherein the T cell modulator is an inhibitor of NOTCH transcription complex, and wherein the T cell modulator is a compound of formula (I) described below.

In a second aspect, the present invention provides a pharmaceutical composition comprising the claimed T cell modulator (TCM) and a pharmaceutically acceptable diluent, excipient, or carrier for use in a method for the prevention, delay of progression or treatment of an autoimmune and inflammatory disease (AIID) in a subject.

In a third aspect, the present invention provides kit of parts comprising a container and a package insert, wherein the container comprises at least one dose of a medicament comprising the claimed T cell modulator (TCM), and the package insert comprises optionally instructions for treating a subject for an autoimmune and inflammatory disease using the medicament.

### Brief description of the figures

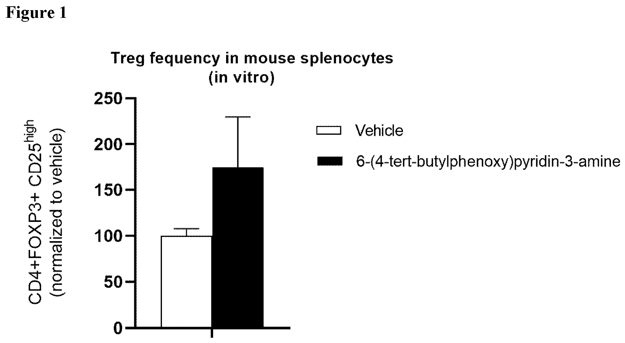

**Figure 1****:** In vitro 6-(4-tert-butylphenoxy)pyridin-3-amine treatment causes an expansion of Treg cells in activated mouse splenocytes. Mouse splenocytes were harvested and single cell suspension stimulated in vitro with anti-CD3 and anti-CD28 antibodies for 96 hours. Treatment with 6-(4-tert-butylphenoxy)pyridin-3-amine, during the same interval of time, caused an expansion of immunosuppressive regulatory T cells (Tregs). Each bar represents the mean ± SD (N=3 replicates).

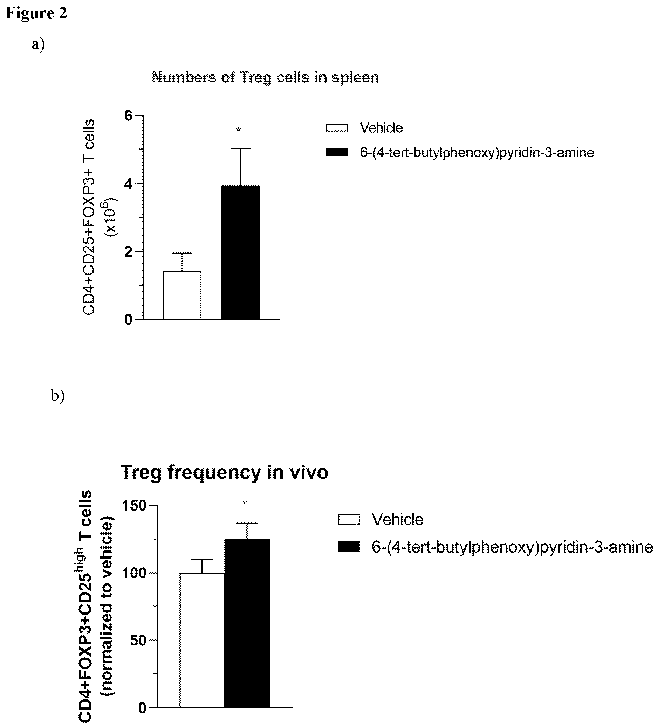

**Figure 2****:** *In vivo* 6-(4-tert-butylphenoxy)pyridin-3-amine dosing leads to an expansion of Treg cells in mouse spleen. C57BL6 wild type mice were treated with 6-(4-tert-butylphenoxy)pyridin-3-amine at 60 mg/kg dose (QD). Treg cells (CD4⁺CD25^{high}FOXP3⁺) number (a) and frequency (b) was significantly increased upon mice dosing with 6-(4-tert-butylphenoxy)pyridin-3-amine. (*) p<0.05. Each bar represents the mean ± SEM (N=3 replicates).

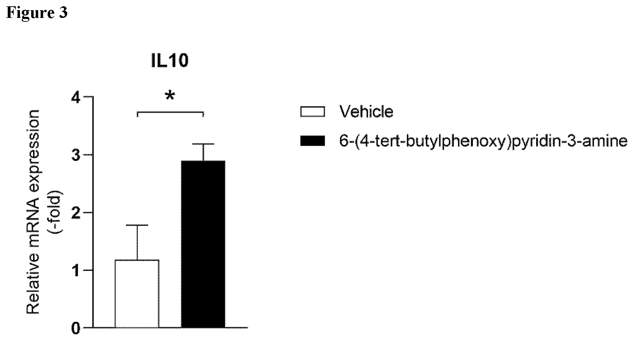

**Figure 3****:** *In vivo* 6-(4-tert-butylphenoxy)pyridin-3-amine dosing leads to an upregulation of anti-inflammatory cytokine IL-10 mRNA in mouse spleen. Quantitative RT-PCR using mRNA extracted from splenocytes in vehicle and 6-(4-tert-butylphenoxy)pyridin-3-amine treated mice showed an upregulation of immune suppressive cytokine IL-10 transcript. Each bar represents the mean ± SD (N=3 replicates). (*) p<0.05

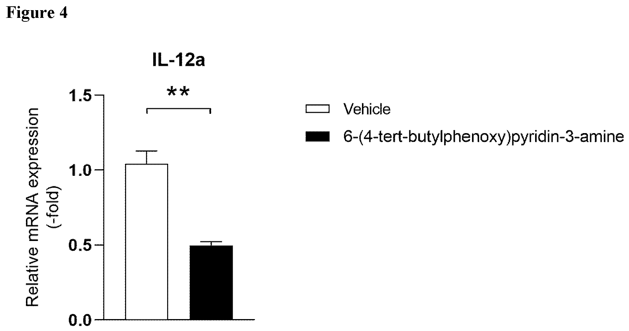

**Figure** 4: *In vivo* 6-(4-tert-butylphenoxy)pyridin-3-amine dosing leads to a downregulation of pro-inflammatory cytokine IL-12a mRNA in mouse spleen. Quantitative RT-PCR using mRNA extracted from splenocytes in vehicle and 6-(4-tert-butylphenoxy)pyridin-3-amine treated mice showed a downregulation of pro-inflammatory cytokine IL-12a transcript. Each bar represents the mean ± SD (N=3 replicates). (**) p <0.01

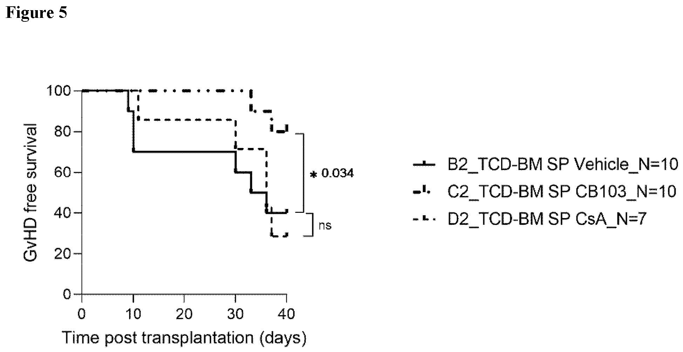

**Figure 5****:** 6-(4-tert-butylphenoxy)pyridin-3-amine prevents development of GvHD. Following allogeneic bone marrow + splenocyte transplantation, mice were treated with vehicle, 6-(4-tert-butylphenoxy)pyridin-3-amine and cyclosporin A. Compared to 40% vehicle treatment and 30% cyclosporin A treatment, 80% of 6-(4-tert-butylphenoxy)pyridin-3-amine animals remained GvHD free.

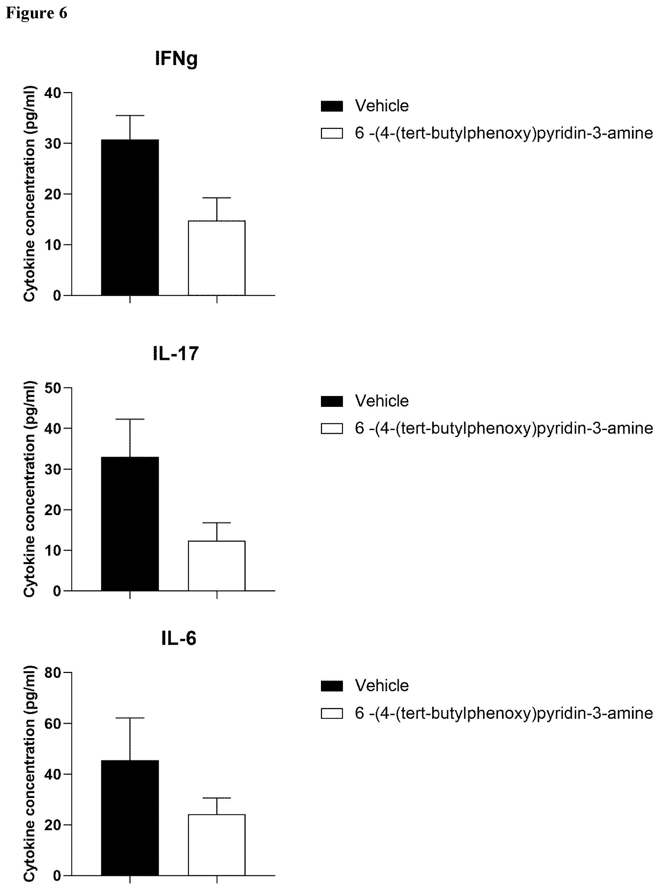

**Figure 6****:** 6-(4-tert-butylphenoxy)pyridin-3-amine downregulates cytokines involved in GvHD. Following allogeneic bone marrow + splenocyte transplantation, mice were treated with vehicle and 6-(4-tert-butylphenoxy)pyridin-3-amine. Compared to vehicle treatment, 6-(4-tert-butylphenoxy)pyridin-3-amine treatment led to a downregulation of IFNG, IL-17 and IL-6.

### Detailed description of the invention

As outlined above, in a first aspect the present invention provides a T cell modulator (TCM) for use in a method for the prevention, delay of progression or treatment of an autoimmune and inflammatory disease (AIID) in a subject, wherein the T cell modulator is an inhibitor of NOTCH transcription complex, and wherein the T cell modulator is a compound of formula (I) described below.

For the purposes of interpreting this specification, the following definitions will apply and whenever appropriate, terms used in the singular will also include the plural and vice versa. It is to be understood that the terminology used herein is for the purpose of describing particular embodiments only and is not intended to be limiting. The terms "comprising", "having", and "including" are to be construed as open-ended terms (i.e., meaning "including, but not limited to,") unless otherwise noted.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "autoimmune and inflammatory disease" also abbreviated herein as (AIID) refers to diseases caused by innate immune cells or adaptive immune cells. Autoimmune and inflammatory diseases as referred herein include alloimmune diseases in which an immune response is triggered against non-self antigens by members of the same species like Graft vs Host disease (GvHD) and allogeneic organ transplant rejection. Innate immune cells are cells of the immune system that are known to be activated by one or more agents (e.g., allergens, chemicals produced upon injury (e.g., opioids and alcohols), polymyxins, crosslinked IgE, crosslinked complement proteins, cytokines produced by T cells or other immune cells (e.g., interferon-y), DAMPs, or PAMPs) that activate downstream signaling pathway(s) in the innate immune cell and result in the activation of one or more functions of the innate immune cell. Adaptive immune cells relate to cells of the adaptive immune system. The adaptive immune system protects higher organisms against infections and other pathological events that may be attributable to foreign substances, using adaptive immune receptors, the antigen-specific recognition proteins that are expressed by hematopoietic cells of the lymphoid lineage and that are capable of distinguishing self from non-self molecules in the host. These lymphocytes may be found in the circulation and tissues of a host, and their recirculation between blood and the lymphatics has been described, including their extravasation via lymph node high endothelial venules, as well as at sites of infection, inflammation, tissue injury and other clinical insults.

The term "autoimmune and inflammatory disease driven by" or "diseases driven by" refers to AIIDs which are caused due to an aberrant activation of cells of immune system (innate or adaptive) against self-antigen or foreign antigen leading to damage to organs. The term "autoimmune and inflammatory disease driven by" or "diseases driven by" have the same meaning as "autoimmune and inflammatory disease caused by" or "diseases caused by" and the respective terms are used synonomously herein.

The term "inflammation" of the "inflammatory disease" refers to one of the biological reactions to harmful stimulants in living tissues, for example, pathogenic microorganisms, damaged cells, stimulants and the like. Inflammation can be caused by a variety of causes, such as infection by microorganisms or injuries, surgery, burns, frostbite, electrical stimulation, and chemicals. The inflammatory disease refers to a disease that involves inflammation as a major lesion. The Inflammatory disease may be selected from the group consisting of sepsis, gastritis, enteritis, nephritis, hepatitis, chronic obstructive pulmonary disease (COPD), pulmonary fibrosis, hypersensitivity colorectal syndrome, inflammatory pain, migraine, headaches, back pain, fibromyalgia, fascial disease, viral infection, bacterial infection, fungal infection, burns, wounds due to surgery, including dental surgery, or accident, prostaglandin excessive syndrome, atherosclerosis, gout, Hodgkin's disease, pancreatitis, conjunctivitis, iritis, sclerotitis, uveitis, and eczema.

The term "autoimmune disease" refers to a disease in which an immune-function abnormality occurs, resulting in immune cells in the body attacking organs or tissues of the body. The autoimmune disease can be categorized as a disease associated with organ-specific autoantibodies and an organ non-specific (systemic) disease. The autoimmune disease may be selected from the group consisting of hemophagocytic lymphohistiocytosis, systemic lupus erythematosus, Kikuchi disease, vasculitis, adult onset Still's disease, rheumatoid arthritis, inflammatory myositis, Behcet disease, IgG4-related disease, Sjogren syndrome, giant cell arteritis, temporal arteritis, Type 1 diabetes, atopic dermatitis, Crohn's disease, systemic sclerosis, psoriasis, multiple sclerosis, and Graves hyperthyroidism.

The term "Graft-versus-Host-Disease (GvHD)" refers to acute, chronic and steroid refractory Graft-versus-Host-Disease (GvHD) and includes graft-versus-host disease (GvHD) in patients undergoing allogenic hematopoietic stem cell (allow-bone marrow) transplantation (allo-HSCT).

The term "T cell modulator" also abbreviated herein as (TCM) refers to a molecule that modulates (e.g., increases or decreases) the numbers, proliferation, viability and/or activity of immune cells.

The term "NOTCH signaling pathway inhibitor" as used herein refers to a compound that is inhibiting the NOTCH signalling pathway. NOTCH signaling pathway inhibitors as used herein include a compound of formula (I) as shown below, a γ-secretase inhibitor, a blocking antibody against NOTCH receptors, a blocking antibody against NOTCH ligands, an agent that blocks trafficking of NOTCH ligands/receptors and an inhibitor of NOTCH transcription complex. The NOTCH signalling pathway represents a critical component in the molecular circuits that control cell fate during development, cell survival and cell proliferation (Shih IeM, Wang TL in Cancer Res 2007;67(5):1879-82). Aberrant activation of this pathway contributes to tumorigenesis. The NOTCH family members are being revealed as oncogenes in an ever-increasing number of cancers. The role of NOTCH in human cancer has been highlighted recently by the presence of activating mutations and amplification of *NOTCH* genes in human cancer and by the demonstration that genes/proteins in the NOTCH signalling pathway could be potential therapeutic targets. It has become clear that one of the major therapeutic targets in the NOTCH pathway are the NOTCH receptors, in which γ-secretase inhibitors prevent the generation of the oncogenic (intracellular) domain of NOTCH molecules and suppress the NOTCH activity. Though significant progress has been made in dissecting the complex workings of this signalling pathway, there are very limited options available for developing novel NOTCH inhibitors. However, the pioneering class of NOTCH inhibitors is already in clinical trials for few cancer types, such as γ-secretase inhibitors AL101 and AL102 from Ayala Pharma (formerly BMS 906024 and BMS-986115 respectively), LY3039478 from Eli Lilly and, PF-03084014 (Nirogacestat) from Springworks Therapeutics, a synthetic small molecule, which inhibits the NOTCH signalling pathway, which may result in induction of growth arrest in tumor cells in which the NOTCH signalling pathway is overactivated.

The term "NOTCH receptors" as used herein refers to the NOTCH receptors NOTCH1, NOTCH2, NOTCH3 and NOTCH4. "A blocking antibody against NOTCH receptors" is a compound specifically binding to the extra-cellular part of the NOTCH receptors hence preventing either constitutive activation of the pathway or activation through ligand binding.

The term "NOTCH ligands" as used herein refers to the NOTCH ligands Delta like 1, Delta like 3, Delta like 4, Jagged, 1, Jagged 2. "A blocking antibody against NOTCH ligands" is a compound specifically binding to one of the ligands hence blocking binding to NOTCH receptors and preventing subsequent activation of the pathway.

The terms "an agent that blocks trafficking of NOTCH ligands/receptors" and "an inhibitor of intracellular trafficking of NOTCH ligands/receptors" which are used herein synonymously refer to a compound that prevents intracellular trafficking of NOTCH ligands or receptors (e.g inhibitors of SERCA2).

The term "inhibitor of NOTCH transcription complex" as used herein refers to a compound that prevents components of NOTCH transcription complex from assembling properly into a functional complex. Non-limiting examples of inhibitors of NOTCH transcription complex include compounds of formula I such as 6-(4-tert-butylphenoxy)pyridin-3-amine, 6-((4'-fluoro-[1,1'-biphenyl]-4-yl)oxy)-2-methylpyridin-3-amine, and N-methyl-6-(4-(thiazol-5-yl)phenoxy)pyridin-3-amine; and compounds such as 2-(2-fluorophenoxy)-4-(1-methyl-1H-pyrazol-5-yl)benzamide and 2-[2-Methoxy-4-[(4-oxo-2-thioxo-5-thiazolidinylidene)methyl]phenoxy]-acetic acid ethyl ester.

The term "gamma secretase inhibitor" (GSI) as used herein refers to a compound that blocks activity of the gamma secretase complex of proteins. Non-limiting examples of gamma secretase inhibitors include AL-101, AL-102, LY3039478, RO4929097, MK-0752, and PF-03084014.

The term "JAK/STAT signaling inhibitor" as used herein refers to inhibitors of JAK1-3 and TYK kinases or inhibitors of downstream mediators of JAK signaling (e.g inhibitors of STAT1-6 proteins). Non-limiting examples of JAK/STAT inhibitors include Ruxolitinib, Itacitinib, PF-06263276, AZD4205, BMS-911543, Ilginatinib, Tofacitinib, Oclacitinib, Fedratinib, Baricitinib, Solcitinib and Abrocitinib.

The term inhibitor of pro-inflammatory cytokines" as used herein refers to inhibitors of IL-6, IL-6R, IFNG, TNFα, IL-17, IL-4 and IL-13. Non-limiting examples include, Siltuximab, Sarilumab, Tocilizumab, Infliximab, Adalimumab, Etanercept, Secukinumab, Ixekizumab, Brodalumab, Omalizumab, Dupilumab, Pascolizumab Anrukinzumab, Lebrikizunab and Tralokinumab.

The term "enhancer of anti-inflammatory cytokines" as used herein refers to IL-10, TGFβ, low dose IL-2 and IL-2 mutein.

The terms "individual," "subject" or "patient" are used herein interchangeably. In certain embodiments, the subject is a mammal. Mammals include, but are not limited to primates (including human and non-human primates). In a preferred embodiment, the subject is a human.

The term "pharmaceutically acceptable diluents, excipients or carriers" as used herein refers to diluents, excipients or carriers that are suitable for use with humans and/or animals without undue adverse side effects (such as toxicity, irritation, and allergic response) commensurate with a reasonable benefit/risk ratio. "Diluents" are agents which are added to the bulk volume of the active agent making up the solid composition. As a result, the size of the solid composition increases, which makes it easier to handle. Diluents are convenient when the dose of drug per solid composition is low and the solid composition would otherwise be too small. "Excipients" can be binders, lubricants, glidants, coating additives or combinations thereof. Thus, excipients are intended to serve multiple purposes. "Carriers" can be solvents, suspending agents or vehicles, for delivering the instant compounds to a subject.

The term "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e. g. an alkaline metal ion, an alkaline earth metal ion, or an aluminum ion; or coordinates with an organic base such as ethanolamine, diethanolamine, triethanolamine, tromethamine, N-methylglucamine, and the like.

The term "about" as used herein refers to +/- 10% of a given measurement.

Thus, in a first aspect the present invention provides a T cell modulator (TCM) for use in a method for the prevention, delay of progression or treatment of an autoimmune and inflammatory disease (AIID) in a subject.

### T cell modulator (TCM)

In one aspect of the disclosure the T cell modulator (TCM) is selected from the group consisting of a NOTCH signaling pathway inhibitor, JAK/STAT signaling inhibitor, inhibitors of pro-inflammatory cytokines and enhancer of anti-inflammatory cytokines.

In one aspect of the disclosure the T cell modulator (TCM) is a NOTCH signaling pathway inhibitor.

In one aspect of the disclosure the T cell modulator (TCM) is a NOTCH signaling pathway inhibitor selected from the group consisting of a γ-secretase inhibitor, a blocking antibody against NOTCH receptors, a blocking antibody against NOTCH ligands, an inhibitor of NOTCH transcription complex and an inhibitor of intracellular trafficking of NOTCH ligands/receptors.

In one aspect of the disclosure the T cell modulator (TCM) is selected from the group consisting of a γ-secretase inhibitor and an inhibitor of NOTCH transcription complex and is preferably an inhibitor of NOTCH transcription complex.

In one aspect of the disclosure the T cell modulator (TCM) is selected from the group consisting of a γ-secretase inhibitor, IMR-1, IMR-1A and a compound of formula (I) as shown below. Preferably the γ-secretase inhibitor is selected from the group consisting of AL-101, AL-102, LY3039478, RO4929097, MK-0752, and PF-03084014, more preferably selected from the group consisting of AL-101, AL-102, PF-03084014 and LY3039478.

In one aspect of the disclosure the T cell modulator (TCM) is selected from the group consisting of a γ-secretase inhibitor and a compound of formula (I) as shown below. Preferably the γ-secretase inhibitor is selected from the group consisting of AL-101, AL-102, LY3039478, RO4929097, MK-0752, and PF-03084014, more preferably selected from the group consisting of AL-101, AL-102, PF-03084014 and LY3039478.

In a further aspect of the disclosure the T cell modulator (TCM) is a γ-secretase inhibitor; or a compound selected from the group consisting of 2-(2-fluorophenoxy)-4-(1-methyl-1H-pyrazol-5-yl)benzamide, 2-[2-Methoxy-4-[(4-oxo-2-thioxo-5-thiazolidinylidene)methyl]phenoxy]-acetic acid ethyl ester, IMR-1, IMR-1A and a compound of formula (I) as shown below.

In a further aspect of the disclosure the T cell modulator (TCM) is a γ-secretase inhibitor; or a compound selected from the group consisting of 2-(2-fluorophenoxy)-4-(1-methyl-1*H*-pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further aspect of the disclosure the T cell modulator (TCM) is a γ-secretase inhibitor selected from the group consisting of AL-101, AL-102, LY3039478, RO4929097, MK-0752, and PF-03084014; or a compound selected from the group consisting of 2-(2-fluorophenoxy)-4-(1-methyl-1H-pyrazol-5-yl)benzamide, 2-[2-Methoxy-4-[(4-oxo-2-thioxo-5-thiazolidinylidene)methyl]phenoxy]-acetic acid ethyl ester, IMR-1, IMR-1A and a compound of formula (I) as shown below.

In a further aspect of the disclosure the T cell modulator (TCM) is a γ-secretase inhibitor selected from the group consisting of AL-101, AL-102, LY3039478, RO4929097, MK-0752, and PF-03084014; or a compound selected from the group consisting of 2-(2-fluorophenoxy)-4-(1-methyl-1H-pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further aspect of the disclosure the T cell modulator (TCM) is a compound selected from the group consisting of of 2-(2-fluorophenoxy)-4-(1-methyl-1H-pyrazol-5-yl)benzamide, 2-[2-Methoxy-4-[(4-oxo-2-thioxo-5-thiazolidinylidene)methyl]phenoxy]-acetic acid ethyl ester, IMR-1, IMR-1A and a compound of formula (I) as shown below.

In a further aspect of the disclosure the T cell modulator (TCM) is a compound selected from the group consisting of 2-(2-fluorophenoxy)-4-(1-methyl-1H-pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further aspect of the disclosure the T cell modulator (TCM) is a γ-secretase inhibitor selected from the group consisting of AL-101, AL-102, LY3039478, RO4929097, MK-0752, and PF-03084014; IMR-1, IMR-1A or a compound of formula (I) as shown below.

In a further aspect of the disclosure the T cell modulator (TCM) is a γ-secretase inhibitor selected from the group consisting of AL-101, AL-102, LY3039478, RO4929097, MK-0752, and PF-03084014; or a compound of formula (I) as shown below.

According to the invention, the T cell modulator (TCM) is a compound of formula (I) 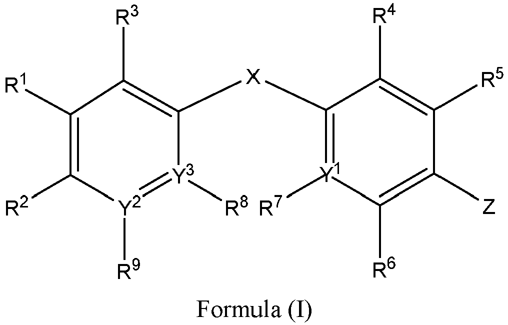
pharmaceutically-acceptable salts, hydrates, solvates, or stereoisomers thereof,
wherein X is selected from CH₂, CF₂, CHF, CO, CHOH, CHO(C₁-C₃) alkyl, NH, N(C₁-C₃ alkyl), S, SO and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl, C₁-C₆ heteroalkyl, C₃-C₁₂ cycloalkyl, C₃-C₁₂ heterocyclyl and C₁-C₆ alkoxy;
wherein R⁴, R⁵ and R⁶ are each independently selected from H, halogen, CN, C₁-C₆ alkoxy, C₁-C₆-S-alkyl, C₁-C₆ alkyl, C₁-C₆ heteroalkyl, C₃-C₁₂ cycloalkyl and C₃-C₁₂ heterocyclyl;
wherein R⁷ is absent when Y¹ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y¹ is C;
wherein R⁸ is absent when Y³ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y³ is C;
wherein R⁹ is absent when Y² is N or is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R¹² is selected from H, NH₂, NHC₁-C₆ alkyl, C₁-C₆ alkyl, C₁-C₆ heteroalkyl, C₂-C₆ alkenyl, C₂-C₆ alkynyl, C₃-C₁₂ cycloalkyl and C₃-C₁₂ heterocyclyl; and optionally one or more pharmaceutically acceptable diluents, excipients or carriers.

In a more preferred embodiment the T cell modulator (TCM) is a compound of formula (I) 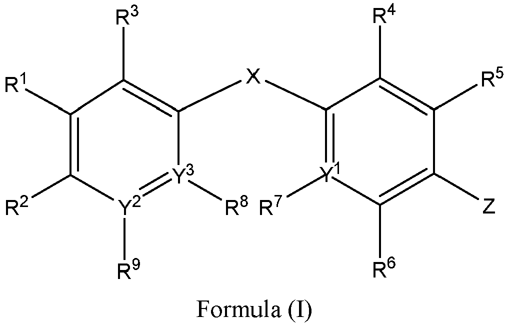
pharmaceutically-acceptable salts, hydrates, solvates, or stereoisomers thereof,
wherein X is selected from CH₂, CF₂, CHF, CO, CHOH, CHO(C₁-C₃) alkyl, NH, N(C₁-C₃ alkyl), S, SO and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl, C₁-C₆ heteroalkyl, C₃-C₁₂ cycloalkyl, C₃-C₁₂ heterocyclyl and C₁-C₆ alkoxy;
wherein R⁴, R⁵ and R⁶ are each independently selected from H, halogen, CN, C₁-C₆ alkoxy, C₁-C₆-S-alkyl, C₁-C₆ alkyl, C₁-C₆ heteroalkyl, C₃-C₁₂ cycloalkyl and C₃-C₁₂ heterocyclyl;
wherein R⁷ is absent when Y¹ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y¹ is C;
wherein R⁸ is absent when Y³ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y³ is C;
wherein R⁹ is absent when Y² is N or is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R¹² is selected from H, NH₂, NHC₁-C₆ alkyl, C₁-C₆ alkyl, C₁-C₆ heteroalkyl, C₂-C₆ alkenyl, C₂-C₆ alkynyl, C₃-C₁₂ cycloalkyl and C₃-C₁₂ heterocyclyl; and optionally one or more pharmaceutically acceptable diluents, excipients or carriers; with the proviso that if R² is C₁-C₆ alkyl or C₃-C₁₂ cycloalkyl, Y¹ is N.

The compounds of formula (I) and their synthesis is described in WO2013093885A1 and WO2020208139A1, respectively.

The term "alkyl" as used herein refers to a saturated straight or branched chain group of carbon atoms derived from an alkane by the removal of one hydrogen atom. C₁-C₃ alkyl comprises for example methyl, ethyl, n-propyl, i-propyl and comprises preferably non-branched C₁-C₃ alkyl. C₁-C₄ alkyl comprises for example methyl, ethyl, n-propyl, i-propyl, n-butyl, i-butyl, tert-butyl and comprises preferably non-branched C₁-C₄ alkyl. C₁-C₆ alkyl comprises for example methyl, ethyl, n-propyl, i-propyl, n-butyl, i-butyl, tert-butyl, n-pentyl, and n-hexyl and comprises preferably non-branched C₁-C₆ alkyl. C₁-C₁₀ alkyl comprises for example methyl, ethyl, n-propyl, i-propyl, n-butyl, i-butyl, tert-butyl, n-pentyl, n-hexyl, n-heptyl, n- octyl, n-nonyl or n-decyl and comprises preferably non-branched C₁-C₁₀ alkyl. The term "C₀ alkyl "as used herein refers to a covalent bond. Thus e.g. the term "C₀ alkylOC₀ alkyl aryl" refers to Oaryl.

The term "C₀-C₃ alkylOC₀-C₃ alkyl aryl" as used herein refers to Oaryl as defined herein when both C₀-C₃ alkyl groups are C₀ alkyl. The term refers to OC₀-C₃ alkyl aryl when the first C₀-C₃ alkyl group is C₀ alkyl. The term refers to C₀-C₃ alkylOaryl when the second C₀-C₃ alkyl group is C₀ alkyl. Preferably C₀-C₃ alkylOC₀-C₃ alkyl aryl is C₀-C₃ alkylOaryl, more preferably Oaryl or C₁-C₃ alkylOaryl. The term "C₀-C₃ alkylOC₀-C₃ alkyl heteroaryl" as used herein refers to Oheteroaryl as defined herein when both C₀-C₃ alkyl groups are C₀ alkyl. The term refers to OC₀-C₃ alkyl heteroaryl when the first C₀-C₃ alkyl group is C₀ alkyl. The term refers to C₀-C₃ alkylOheteroaryl when the second C₀-C₃ alkyl group is C₀ alkyl. Preferably C₀-C₃ alkylOC₀-C₃ alkyl heteroaryl is C₀-C₃ alkylOheteroaryl, more preferably Oheteroaryl or C₁-C₃ alkylOheteroaryl, most preferably Oheteroaryl. The aryl and the heteroaryl of C₀-C₃ alkylOC₀-C₃ alkyl aryl and C₀-C₃ alkylOC₀-C₃ alkyl heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably are optionally substituted by NH₂.

The term "heteroalkyl" as used herein refers to an alkyl radical as defined herein wherein one, two, three or four hydrogen atoms have been replaced with a substituent independently selected from the group consisting of OR^{a}, C(O)OR^{a}, NR^{b}R^{c}, C(O)NR^{b}R^{c}, S(O)ₙR^{d} (where n is an integer from 0 to 2) and halogen, with the understanding that the point of attachment of the heteroalkyl radical is through a carbon atom, wherein R^{a} is H, C₁-C₃ alkylcarbonyl, C₁-C₃ alkyl, or C₃₋₇ cycloalkyl; R^{b} and R^{c} are each independently H, C₁-C₃ alkylcarbonyl, C₁-C₃ alkyl, C₃₋₇ cycloalkyl or NR^{b}R^{c} is guanidinyl; and when n is 0, R^{d} is H, C₁-C₃ alkyl or C₃₋₇ cycloalkyl, and when n is 1 or 2, R^{d} is C₁-C₃ alkyl or C₃₋₇ cycloalkyl. Preferably. the term "heteroalkyl" or "heteroalkanediyl" as used herein refers to an alkyl radical or an alkanediyl radical as defined herein wherein one, two, three or four hydrogen atoms have been replaced with a substituent independently selected from the group consisting of OH, NH₂, guanidinyl and halogen, more preferably wherein one or two hydrogen atoms have been replaced with a substituent independently selected from the group consisting of OH, NH₂ and halogen. Representative examples include, but are not limited to, 2-hydroxyethyl, 2-hydroxypropyl, 3-hydroxypropyl, 2-hydroxy-1-hydroxymethylethyl, 2-hydroxy-1-methylethyl, 2,3-dihydroxypropyl, 1-hydroxymethylethyl, 3-hydroxybutyl, 2,3-dihydroxybutyl, 1-hydroxy-2-methylpropyl, 3-hydroxy-1-(2-hydroxyethyl)-propyl, 2-hydroxy-1-methylpropyl, 1,1,1-trifluoroethyl, 1,1,1-trifluoromethyl, 2,2,3,3-tetrafluoropropyl.

The term " C₃₋₁₂ cycloalkyl " and "C₃₋₇ cycloalkyl" as used herein refers to a monocyclic, bicyclic, tricyclic or tetracyclic hydrocarbon group, usually to a monovalent saturated monocyclic or bicyclic hydrocarbon group, preferably a monovalent saturated monocyclic goup of 3-12 or 3-7 carbons, respectively derived from a cycloalkane by the removal of a single hydrogen atom. "C₃₋₇ cycloalkyl" includes cyclopropyl, cyclobutyl, cyclopentyl, cyclohexyl and cycloheptyl. The term "C₃₋₁₂ cycloalkyl " and "C₃₋₇ cycloalkyl" as used herein also includes cycloalkyl groups that comprise a C₁₋₃ alkyl radical. Examples of such "C₃₋₇ cycloalkyl" groups comprise cyclopropylmethyl, 2-cyclopropylethyl, cyclobutylmethyl, 2-cyclobutylethyl, cyclopentylmethyl, 2-cyclopentylethyl. Cycloalkyl groups of this invention can be optionally substituted.

The term "aryloxy" or "Oaryl" which are used interchangeably herein refers to a radical -OR where R is an aryl as defined herein, e. g. phenoxy.

The term "C₁-C₆ alkoxy" or "OC₁-C₆ alkyl" which are used interchangeably herein refers to a radical -OR where R is a C₁-C₆ alkyl as defined herein. Examples are methoxy, ethoxy, propoxy, butoxy.

The term "aryl" as used herein refers to a mono- or bicyclic carbocyclic ring system having one or two aromatic rings, and is preferably a monocyclic carbocyclic ring system. The aryl group can also be fused to a cyclohexane, cyclohexene, cyclopentane, or cyclopentene ring or to a cyclohexane, cyclohexene, cyclopentane, or cyclopentene ring comprising a carbonyl group. The aryl groups of this invention can be optionally substituted as further described below. A preferred aryl group and optionally substituted aryl group, respectively of this invention is a phenyl group or substituted phenyl group. Substituents can be e.g. NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹².

The term "heteroaryl" as used herein refers to substituted and unsubstituted aromatic 5-, or 6-membered monocyclic groups and 9- or 10-membered bicyclic groups, preferably a substituted and unsubstituted aromatic 5-, or 6- membered monocyclic group, which have at least one heteroatom (O, S or N) in at least one of the ring(s). Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic group may contain only carbon atoms and may be saturated, partially saturated, or unsaturated. Heteroaryl groups must include at least one fully aromatic ring but the other fused ring or rings may be aromatic or non-aromatic. The heteroaryl group may be attached at any available nitrogen or carbon atom of any ring. Heteroaryl groups of this invention can be optionally substituted as further described below. Usually, a heteroaryl group and optionally substituted heteroaryl group, respectively of this invention is selected from the group consisting of substituted and/or unsubstituted aromatic 5-, or 6- membered monocyclic groups, which have at least one heteroatom (O, S or N), preferably one or two heteroatoms selected from S and N in the ring, more preferably one S and one N in the ring, or one or two N in the ring. A preferred heteroaryl group is an optionally substituted heteroaryl group, selected from the group consisting of an optionally substituted pyridinyl group, an optionally substituted pyrimidinyl group, an optionally substituted di- or triazine group, an optionally substituted thiazole group, an optionally substituted oxazole group, and an optionally substituted imidazole group. An even more preferred heteroaryl group is an optionally substituted pyridinyl group, an optionally substituted pyrimidinyl group, an optionally substituted imidazole group or an optionally substituted thiazole group. Most preferably an optionally substituted pyridinyl group, an optionally substituted imidazole group or an optionally substituted thiazole group, is used as heteroaryl group in the present invention. Optional substituents can be e.g. NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹², or NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl.

The term "heterocyclyl" as used herein means a saturated, monocyclic ring with 3 to 12, preferably with 3 to 7, more preferably 5 to 6 ring atoms which contains up to 3, preferably 1 or 2 heteroatoms selected independently from nitrogen, oxygen or sulfur, and wherein the remaining ring atoms being carbon atoms. Examples of such saturated heterocycles include [1,3]dioxanyl, [1,3]dioxolanyl, pyrrolidinyl, morpholinyl, piperazinyl, piperidinyl, oxazolidinyl, thiazolidinyl, azepanyl and the like. Preferably such heterocyclyl groups are unsubstituted. The terms "halo" or "halogen" as used herein refers to F, Cl, Br, or I and is preferably F, Cl, or Br, more preferably F.

The term "optionally substituted" or "substituted" means that the referenced group is substituted with one or more additional group(s), preferably with one additional group, individually and independently selected from the listed groups.

AL101 (not part of the invention) (formerly BMS 906024), which has the chemical name (2R,3S)-N1-((S)-1-methyl-2-oxo-5-phenyl-2,3-dihydro-1H-benzo[e][1,4]diazepin-3-yl)-2,3-bis(3,3,3-trifluoropropyl)succinamide is described e.g. in WO2012129353A1 or NCT03691207 and is represented by the structural formula indicated below:

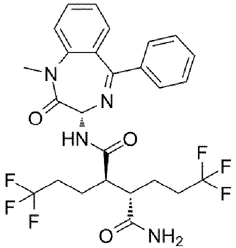

AL102 (not part of the invention) (BMS 986115), which has the chemical name (2S,3R)-N-[(3S)-5-(3-fluorophenyl)-9-methyl-2-oxo-1,3-dihydro-1,4-benzodiazepin-3-yl]-2,3-bis(3,3,3-trifluoropropyl)butanediamide is described e.g. in WO2014047372A1and is represented by the structural formula indicated below:

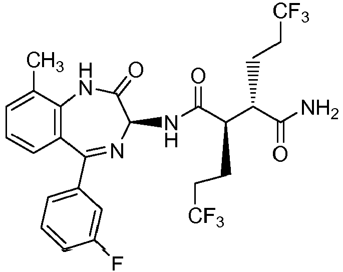

LY3039478 (not part of the invention) which has the chemical name 4,4,4-trifluoro-*N*-[(2*S*)-1-[[(7*S*)-5-(2-hydroxyethyl)-6-oxo-7*H*-pyrido[2,3-d][3]benzazepin-7-yl]amino]-1-oxopropan-2-yl]butanamide is described e.g. in Massard C et al.. Ann Oncol. 2018;29(9):1911-1917 or in WO2020131998A1 and is represented by the structural formula indicated below:

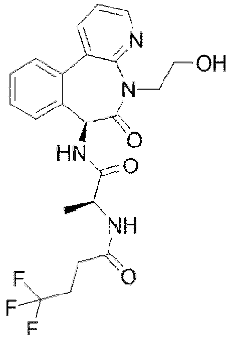

RO4929097 (not part of the invention) which has the chemical name 2,2-dimethyl-N-[(7S)-6-oxo-5,7-dihydrobenzo[d][1]benzazepin-7-yl]-*N*'-(2,2,3,3,3-pentafluoropropyl)propanediamide is described e.g. in Huynh C et al. PLoS One . 2011;6(9) or in WO2020131998A1and is represented by the structural formula indicated below:

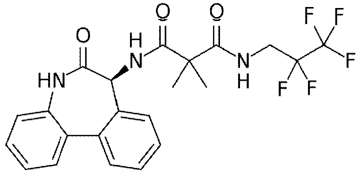

MK-0752 (not part of the invention) which has the chemical name 3-[4-(4-chlorophenyl)sulfonyl-4-(2,5-difluorophenyl)cyclohexyl]propanoic acidis described e.g. in US2004116404A1and is represented by the structural formula indicated below:

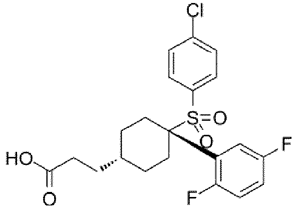

PF-03084014 (not part of the invention) which has the chemical name (2*S*)-2-[[(2*S*)-6,8-difluoro-1,2,3,4-*tetrahydronaphthalen-2-yl]amino]-N-[1-[1-(2,2-dimethylpropylamino)-2-methylpropan-2-*yl]imidazol-4-yl]pentanamideis described e.g. in US7342118, US7795447 and US7951958 and is represented by the structural formula indicated below:

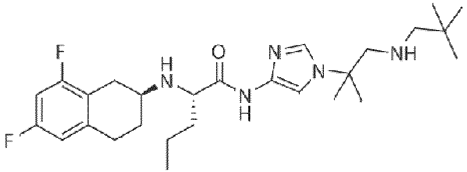

2-(2-fluorophenoxy)-4-(1-methyl-1H-pyrazol-5-yl)benzamide also referred to as RBPJ INhibitor1 (RIN1) is described e.g. in Hurtado C et al., 2019 Sci Rep 9, 10811 and is represented by the structural formula indicated below:

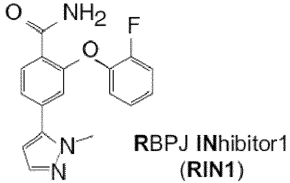

2-[2-Methoxy-4-[(4-oxo-2-thioxo-5-thiazolidinylidene)methyl]phenoxy]-acetic acid ethyl ester ) is obtainable from e.g. Sigma Aldrich and is represented by the structural formula indicated below:

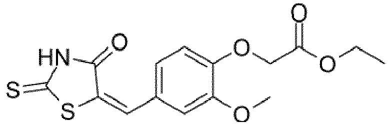

IMR-1 (not part of the invention) is described e.g. in Astudillo L. et al., 2016, Cancer Res Jun 15;76(12):3593-603 and is represented by the structural formula indicated below:

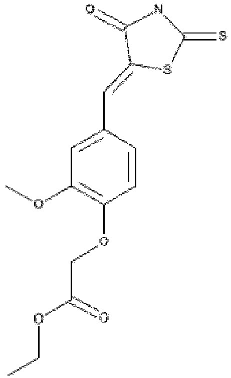

IMR-1A (not part of the invention) is described e.g. in Astudillo L. et al., 2016, Cancer Res Jun 15;76(12):3593-603 and is represented by the structural formula indicated below:

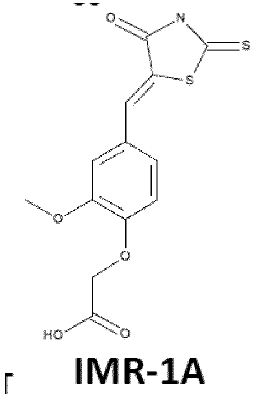

"6-(4-Tert-Butylphenoxy)Pyridin-3-Amine" is represented by the structural formula indicated below:

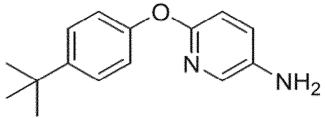

6-(4-Tert-Butylphenoxy)Pyridin-3-Amine is a synthetic small molecule (Molecular Mass: 242.32 g/mol) and is described e.g. in WO2013093885A1.

The present invention also encompasses chemical modifications of the compounds of the present invention to prolong their circulating lifetimes. Non-limiting examples of methods for transiently, or reversibly, pegylating drugs, including polypeptide-based drugs, are provided in U.S. Pat. Nos. 4,935,465 (issued in Jun. 19, 1990) and 6,342,244 (issued Jan. 29, 2002); and in U.S. published applications number US2006/0074024. One skilled in the art would typically find more details about PEG-based reagents in, for example, published applications WO2005047366, US2005171328, and those listed on the NEKTAR PEG Reagent Catalog^{®} 2005-2006 (Nektar Therapeutics, San Carlos, Calif.).

The invention also relates to salts, hydrates or solvates of the compounds of the present invention. Preferably, these salts, hydrates and/or solvates are pharmaceutically acceptable.

The invention also relates to stereoisomers of the compounds of formula (I). "Stereoisomer" or "stereoisomers" refer to compounds that differ in the chirality of one or more stereocentres. Stereoisomers include enantiomers and diastereomers. A compound of formula (I) may exist in stereoisomeric form if they possess one or more asymmetric centres or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (see discussion in Chapter 4 of Advanced Organic Chemistry, 4th ed., J. March, John Wiley and Sons, New York, 1992).

A skilled person will know that, if the compounds of the present invention contain charged group, a suitable counterion will be derived from an organic or inorganic acid. Such counterions include halide (such as chloride, bromide, fluoride, iodide), sulfate, phosphate, acetate, succinate, citrate, lactate, maleate, fumarate, palmitate, cholate, glutamate, glutarate, tartrate, stearate, salicylate, methanesulfonate, benzenesulfonate, sorbate, picrate, benzoate, cinnamate, and the like. If the polar moiety is a negatively charged group, a suitable counterion will be selected from sodium, ammonium, barium, calcium, copper, iron, lithium, potassium and zinc, and the like.

When R¹ is C₀-C₃ alkylOC₀-C₃ alkyl aryl or C₀-C₃ alkylOC₀-C₃ alkyl heteroaryl, the optional substitutions of the aryl and the heteroaryl group are preferably in para position.

When R² is aryl or heteroaryl, the optional substitutions are preferably in ortho or meta position, provided that the substitutents are not halogen, OC₁-C₆ alkyl or methyl and are in para position when the substituents are halogen, OC₁-C₆ alkyl or methyl.

When R⁹ is C₀-C₃ alkylOC₀-C₃ alkyl aryl or C₀-C₃ alkylOC₀-C₃ alkyl heteroaryl the optional substitutions of the aryl and the heteroaryl group are preferably in para position.

In one embodiment X is selected from CH₂, CF₂, CHF, NH, N(C₁-C₃ alkyl), S, SO and O. In a further embodiment X is selected from CO, CHOH, CHO(C₁-C₃) alkyl, S, SO and O. In a preferred embodiment X is selected from CH₂, NH, and O. In a more preferred embodiment X is selected from CH₂, CO, CHOH, CHO(C₁-C₃) alkyl, NH, and O. In an even more preferred embodiment X is selected from CH₂, CO, CHOH, CHO(C₁-C₃) alkyl, and O. In a particular preferred embodiment X is selected from CH₂, CO, CHOH, CHOCH₃, and O. In a more particular preferred embodiment X is selected from CH₂ and O. In an even more particular preferred embodiment X is O.

In one embodiment R¹ is selected from H, halogen, C₁-C₆ alkyl, C₁-C₆ heteroalkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further embodiment R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl. In a preferred embodiment R¹ is selected from H, halogen and C₁-C₄ alkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further preferred embodiment R¹ is selected from H, halogen, C₁-C₆ alkyl and C₁-C₆ heteroalkyl. In a more preferred embodiment R¹ is selected from H, C₁-C₆ alkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted NH₂; C₀-C₃ alkylOC₀-C₃ alkyl heteroaryl wherein the heteroaryl is optionally substituted by NH₂, C₁-C₆ alkyl, C₁-C₆ heteroalkyl, halogen, CN, preferably is optionally substituted by NH_{2.} In an even more preferred embodiment R¹ is selected from H, C₁-C₄ alkyl and C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further more preferred embodiment R¹ is selected from H, halogen and C₁-C₄ alkyl. In an even more preferred embodiment R¹ is selected from H, methyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from H and methyl.

In one embodiment R² is selected from C₁-C₆ alkyl, C₃-C₁₂ cycloalkyl, aryl and heteroaryl wherein the aryl and the heteroaryl is substituted by a substituent selected from NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹² and C₁-C₆ alkyl C(O)R¹². In a further embodiment R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C(O)R¹², C₁-C₆ alkyl C(O)R¹² .

In a preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C₃-C₁₂ cycloalkyl, C₃-C₁₂ heterocyclyl, C(O)R¹² and C₁-C₆ alkyl C(O)R¹². In a more preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹². In a further more preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C₃-C₁₂ cycloalkyl, C₃-C₁₂ heterocyclyl. In a particular preferred embodiment R² is selected from C₁-C₆ alkyl, C₃-C₁₂ cycloalkyl, aryl and heteroaryl wherein the aryl and the heteroaryl are optionally substituted by C₁-C₆ alkyl, halogen, C₁-C₆ heteroalkyl, C(O)R¹², C₁-C₆ alkyl C(O)R¹². In a further particular preferred embodiment R² is selected from C₁-C₆ alkyl, C₃-C₁₂ cycloalkyl, aryl and heteroaryl wherein the aryl and the heteroaryl are optionally substituted by C₁-C₆ alkyl, halogen. In a further particular preferred embodiment R² is selected from C₁-C₆ alkyl, C₃-C₁₂ cycloalkyl, aryl and heteroaryl wherein the aryl and the heteroaryl are optionally substituted by NH₂, halogen, CN. In a further particular preferred embodiment R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹². Among the particular preferred embodiments, the embodiment wherein R² is selected from aryl and heteroaryl wherein the aryl and the heteroaryl are optionally substituted by C₁-C₆ alkyl, halogen, C₁-C₆ heteroalkyl, C(O)R¹², C₁-C₆ alkyl C(O)R¹² is preferred. In an even more particular preferred embodiment R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹².

In an even more particular preferred embodiment R² is selected from C₁-C₆ alkyl, C₃-C₁₂ cycloalkyl, phenyl, pyridyl, imidazole and thiazole, wherein the phenyl, pyridyl, imidazole and thiazole each are optionally substituted by NH₂, OC₁-C₆ alkyl, C₁-C₆ alkyl, C₁-C₆ heteroalkyl, halogen, CN, C(O)R¹², C₁-C₆ alkyl C(O)R¹². In a further even more particular preferred embodiment R² is selected from C₁-C₆ alkyl, C₃-C₁₂ cycloalkyl, phenyl, thiazole, pyridyl and imidazole wherein the phenyl, thiazole, pyridyl and imidazole each are optionally substituted by NH₂, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by NH₂, C₁-C₆ alkyl, C₁-C₆ heteroalkyl, halogen, CN, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen, C₁-C₆ heteroalkyl, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by NH₂, halogen, CN. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C(O)R¹², C₁-C₆ alkyl C(O)R¹². Among the even more particular preferred embodiments the embodiment wherein R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen, C₁-C₆ heteroalkyl, C(O)R¹², C₁-C₆ alkyl C(O)R¹² is preferred. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each optionally substituted by C(O)R¹², C₁-C₆ alkyl C(O)R¹². In a further even more particular preferred R² is selected from C₁-C₆ alkyl, C₃-C₁₂ cycloalkyl, phenyl, pyridyl, imidazole and thiazole, wherein the phenyl, pyridyl, imidazole and thiazole each are optionally substituted by OC₁-C₆ alkyl, C₁-C₆ alkyl, C₁-C₆ heteroalkyl, halogen, C₁-C₆ alkyl C(O)R¹². In a most preferred embodiment R² is C₁-C₆ alkyl, more preferably tert-butyl.

In a preferred embodiment when R² is heteroaryl the heteroaryl is an optionally substituted aromatic 5-, or 6- membered monocyclic group.

In one embodiment R³ is selected from H, halogen, C₁-C₆ alkyl, and C₃-C₁₂ cycloalkyl. In a preferred embodiment R³ is selected from H, halogen, C₁-C₄ alkyl, and C₃-C₇ cycloalkyl. In a more preferred embodiment R³ is selected from H, halogen and C₁-C₄ alkyl. In an even more preferred embodiment R³ is H.

In one embodiment R⁴, R⁵ and R⁶ are each independently selected from H, halogen, CN, C₁-C₆ alkyl and C₁-C₆ heteroalkyl. In a preferred embodiment R⁴, R⁵ and R⁶ are each independently selected from H, halogen, CN, C₁-C₄ alkyl and C₁-C₄ heteroalkyl. In an even more preferred embodiment R⁴, R⁵ and R⁶ are each independently selected from H, halogen, and C₁-C₄ alkyl. In a particular preferred embodiment R⁴, R⁵ and R⁶ are each independently selected from H, halogen, and methyl. In a more particular preferred embodiment R⁴ is selected from H and halogen and/or R⁵ and/or R⁶ are selected from H and C₁-C₆ alkyl, in particular from H and C₁-C₄ alkyl, more particular from H and methyl.

In one embodiment R⁷ is absent when Y¹ is N or is selected from H, halogen, C₁-C₆ alkyl and C₃-C₁₂ cycloalkyl when Y¹ is C. In a preferred embodiment R⁷ is absent when Y¹ is N or is selected from H, halogen, C₁-C₄ alkyl and C₃-C₇ cycloalkyl when Y¹ is C. In a more preferred embodiment R⁷ is absent when Y¹ is N or is selected from H, halogen and C₁-C₄ alkyl, preferably selected from H, halogen, and methyl when Y¹ is C. In an even more preferred embodiment R⁷ is absent when Y¹ is N or is selected from H, halogen, and methyl when Y¹ is C. In a particular preferred embodiment R⁷ is absent when Y¹ is N or is selected from H and halogen when Y¹ is C. In a more particular preferred embodiment R⁷ is absent.

In one embodiment R⁸ is absent when Y³ is N or is selected from H, halogen, C₁-C₆ alkyl and C₃-C₁₂ cycloalkyl when Y³ is C. In a preferred embodiment R⁸ is absent when Y³ is N or is selected from H, halogen, C₁-C₄ alkyl and C₃-C₇ cycloalkyl when Y³ is C. In a more preferred embodiment R⁸ is absent when Y³ is N or is selected from H, halogen and C₁-C₄ alkyl preferably selected from H, halogen, and methyl when Y³ is C. In an even more preferred embodiment R⁸ is absent when Y³ is N or is selected from H, halogen, and methyl when Y³ is C. In a particular preferred embodiment R⁸ is absent when Y³ is N or is selected from H and halogen when Y³ is C. In a particular preferred embodiment R⁸ is H.

In one embodiment R⁹ is absent when Y² is N or is selected from H, halogen, C₁-C₆ alkyl and C₃-C₁₂ cycloalkyl when Y² is C. In a preferred embodiment R⁹ is absent when Y² is N or is selected from H, halogen, C₁-C₄ alkyl and C₃-C₇ cycloalkyl when Y² is C. In a more preferred embodiment R⁹ is absent when Y² is N or is selected from H and C₁-C₄ alkyl preferably selected from H, halogen, and methyl when Y² is C. In an even more preferred embodiment R⁹ is absent when Y² is N or is selected from H, halogen, and methyl when Y² is C. In a particular preferred embodiment R⁹ is absent when Y² is N or is selected from H and halogen, preferably H, when Y² is C.

In one embodiment R⁷ is absent when Y¹ is N or is selected from H, halogen, C₁-C₆ alkyl and C₃-C₁₂ cycloalkyl when Y¹ is C, preferably selected from H, halogen, and methyl when Y¹ is C, R⁸ is absent when Y³ is N or is selected from H, halogen, C₁-C₆ alkyl and C₃-C₁₂ cycloalkyl, preferably selected from H, halogen, and methyl when Y³ is C, and R⁹ is absent when Y² is N or is selected from H, halogen, C₁-C₆ alkyl and C₃-C₁₂ cycloalkyl, preferably selected from H, halogen, and methyl when Y² is C.

In a preferred embodiment R⁷ is absent when Y¹ is N or is selected from H and halogen when Y¹ is C, R⁸ is absent when Y³ is N or is H when Y³ is C, and R⁹ is absent when Y² is N or is selected from H and methyl when Y² is C.

In one embodiment at least one of R¹⁰ and R¹¹ is C₁-C₆ alkyl, preferably C₁-C₄ alkyl, more preferably methyl. In a preferred embodiment R¹⁰ and R¹¹ are independently selected from H and methyl. In a more preferred embodiment R¹⁰ is H and R¹¹ is selected from H and C₁-C₄ alkyl. In an even more preferred embodiment R¹⁰ is H and R¹¹ is H or methyl. In a particular preferred embodiment R¹⁰ is H and R¹¹ is C₁-C₆ alkyl. In a more particular preferred embodiment R¹⁰ is H and R¹¹ is C₁-C₄ alkyl. In an even more particular preferred embodiment R¹⁰ is H and R¹¹ is methyl.

In one embodiment R¹² is selected from NH₂, NHC₁-C₆ alkyl, C₁-C₆ alkyl, and C₁-C₆ heteroalkyl. In a preferred embodiment R¹² is selected from NH₂, C₁-C₆ alkyl, and C₁-C₆ heteroalkyl. In a more preferred embodiment R¹² is NH₂.

In one embodiment Y¹ is N. In a preferred embodiment Y¹ and Y² are each independently selected from N and C and Y³ is C. In a more preferred embodiment Y¹ is selected from N and C and Y² and Y³ are selected from N and C with the proviso that one of Y²and Y³ is C. In a particular preferred embodiment Y¹ is N and R⁷ is absent, Y² is selected from N and C and Y³ is C. In a more particular preferred embodiment Y¹ is N and R⁷ is absent.

In one embodiment at least one of the substituents selected from R¹, R³, R⁴, R⁵, R⁶, R⁷, R⁸, and R⁹ is not H. In a further embodiment at least one of the substituents selected from R¹, R³, R⁸, and R⁹ is not H.

Preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl; preferably with the proviso that if R² is C₁-C₆ alkyl or C₃-C₁₂ cycloalkyl, Y¹ is N.

Further preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl; with the proviso that when R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, R² is selected from aryl and heteroaryl wherein the aryl and the heteroaryl are optionally substituted by NH₂, halogen, CN, preferably wherein the aryl and the heteroaryl are not substituted; preferably with the further proviso that if R² is C₁-C₆ alkyl or C₃-C₁₂ cycloalkyl, Y¹ is N.

More preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl, with the proviso that when R² is selected from aryl and heteroaryl wherein the aryl and the heteroaryl are substituted by C₁-C₆ alkyl, halogen, C₁-C₆ heteroalkyl, C(O)R¹², C₁-C₆ alkyl C(O)R¹², R¹ is selected from H, halogen, C₁-C₆ alkyl and C₁-C₆ heteroalkyl, preferably is H or methyl; preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those ,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, CN;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C;
preferably with the proviso that if R² is C₁-C₆ alkyl or C₃-C₁₂ cycloalkyl, Y¹ is N.

Even more preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₄ alkyl and C₁-C₄ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₄ alkyl and C₃-C₇ cycloalkyl;
wherein R⁴, R⁵ and R⁶ are each independently selected from H, halogen, CN, C₁-C₄ alkyl and C₁-C₄ heteroalkyl;
wherein R⁷ is absent when Y¹ is N or is selected from H, halogen, C₁-C₄ alkyl and C₃-C₇ cycloalkyl when Y¹ is C;
wherein R⁸ is absent when Y³ is N or is selected from H, halogen, C₁-C₄ alkyl and C₃-C₇ cycloalkyl when Y³ is C;
wherein R⁹ is absent when Y² is N or is selected from H, halogen, C₁-C₄ alkyl and C₃-C₇ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, more preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, C₁-C₆ heteroalkyl, halogen, C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ heteroalkyl;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, halogen;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂, wherein R¹ is preferably selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN, preferably optionally substituted by halogen, C₁-C₆ alkyl,
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R1²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl; with the proviso that when R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, R² is selected from aryl and heteroaryl wherein the aryl and the heteroaryl are optionally substituted by NH₂, halogen, CN, preferably wherein the aryl and the heteroaryl are not substituted; preferably with the further proviso that if R² is C₁-C₆ alkyl or C₃-C₁₂ cycloalkyl, Y¹ is N.

More preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl, with the proviso that when R² is selected from aryl and heteroaryl wherein the aryl and the heteroaryl are substituted by C₁-C₆ alkyl, halogen, C₁-C₆ heteroalkyl, C(O)R¹², C₁-C₆ alkyl C(O)R¹², R¹ is selected from H, halogen, C₁-C₆ alkyl and C₁-C₆ heteroalkyl, preferably is H or methyl;
preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more preferred compounds of formula (I) pharmaceutically-acceptable salts, hydrates, solvates, or stereoisomers thereof are those ,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, CN;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C;
preferably with the proviso that if R² is C₁-C₆ alkyl or C₃-C₁₂ cycloalkyl, Y¹ is N.

Even more preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₄ alkyl and C₁-C₄ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₄ alkyl and C₃-C₇ cycloalkyl;
wherein R⁴, R⁵ and R⁶ are each independently selected from H, halogen, CN, C₁-C₄ alkyl and C₁-C₄ heteroalkyl;
wherein R⁷ is absent when Y¹ is N or is selected from H, halogen, C₁-C₄ alkyl and C₃-C₇ cycloalkyl when Y¹ is C;
wherein R⁸ is absent when Y³ is N or is selected from H, halogen, C₁-C₄ alkyl and C₃-C₇ cycloalkyl when Y³ is C;
wherein R⁹ is absent when Y² is N or is selected from H, halogen, C₁-C₄ alkyl and C₃-C₇ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, more preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, C₁-C₆ heteroalkyl, halogen, C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ heteroalkyl;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, halogen;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂, wherein R¹ is preferably selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN, preferably optionally substituted by halogen, C₁-C₆ alkyl,
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C;
preferably with the proviso that if R² is C₁-C₆ alkyl or C₃-C₁₂ cycloalkyl, Y¹ is N.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

More particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁸ is selected from H, halogen, C₁-C₆ alkyl and C₃-C₁₂ cycloalkyl;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl;
preferably with the proviso that if R² is C₁-C₆ alkyl or C₃-C₁₂ cycloalkyl, Y¹ is N.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, C₁-C₆ alkyl, C₀-C₃ alkylOC₀-C₃ alkyl aryl wherein the aryl is
optionally substituted by NH₂; C₀-C₃ alkylOC₀-C₃ alkyl heteroaryl wherein the heteroaryl is optionally substituted by NH₂;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁷ is absent when Y¹ is N or is H when Y¹ is C;
wherein R⁸ is absent when Y³ is N or is H when Y³ is C;
wherein R⁹ is absent when Y² is N or is H when Y² is C; and
wherein R¹² is NH₂; preferably with the proviso that if R² is C₁-C₆ alkyl or C₃-C₁₂ cycloalkyl, Y¹ is N.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, C₁-C₆ alkyl, C₀-C₃ alkylOC₀-C₃ alkyl aryl wherein the aryl is optionally substituted by NH₂; C₀-C₃ alkylOC₀-C₃ alkyl heteroaryl wherein the heteroaryl is optionally substituted by NH₂;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂; preferably with the proviso that if R² is C₁-C₆ alkyl or C₃-C₁₂ cycloalkyl, Y¹ is N.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁷ is absent when Y¹ is N or is H when Y¹ is C;
wherein R⁸ is absent when Y³ is N or is H when Y³ is C;
wherein R⁹ is absent when Y² is N or is H when Y² is C; and
wherein R¹² is NH₂; preferably with the proviso that if R² is C₁-C₆ alkyl or C₃-C₁₂ cycloalkyl, Y¹ is N.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Further even more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, and is preferably O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl and C₃-C₁₂ cycloalkyl, and is preferably C₁-C₆ alkyl;
wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂.

Even more particular preferred compounds of the compound of formula (I) are selected from the group consisting of

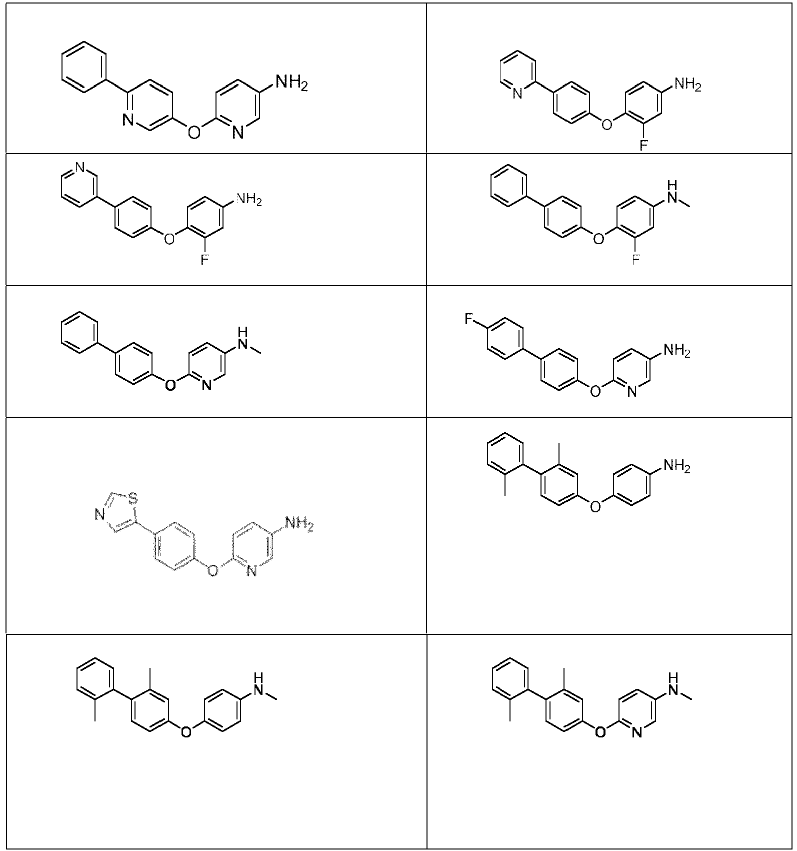

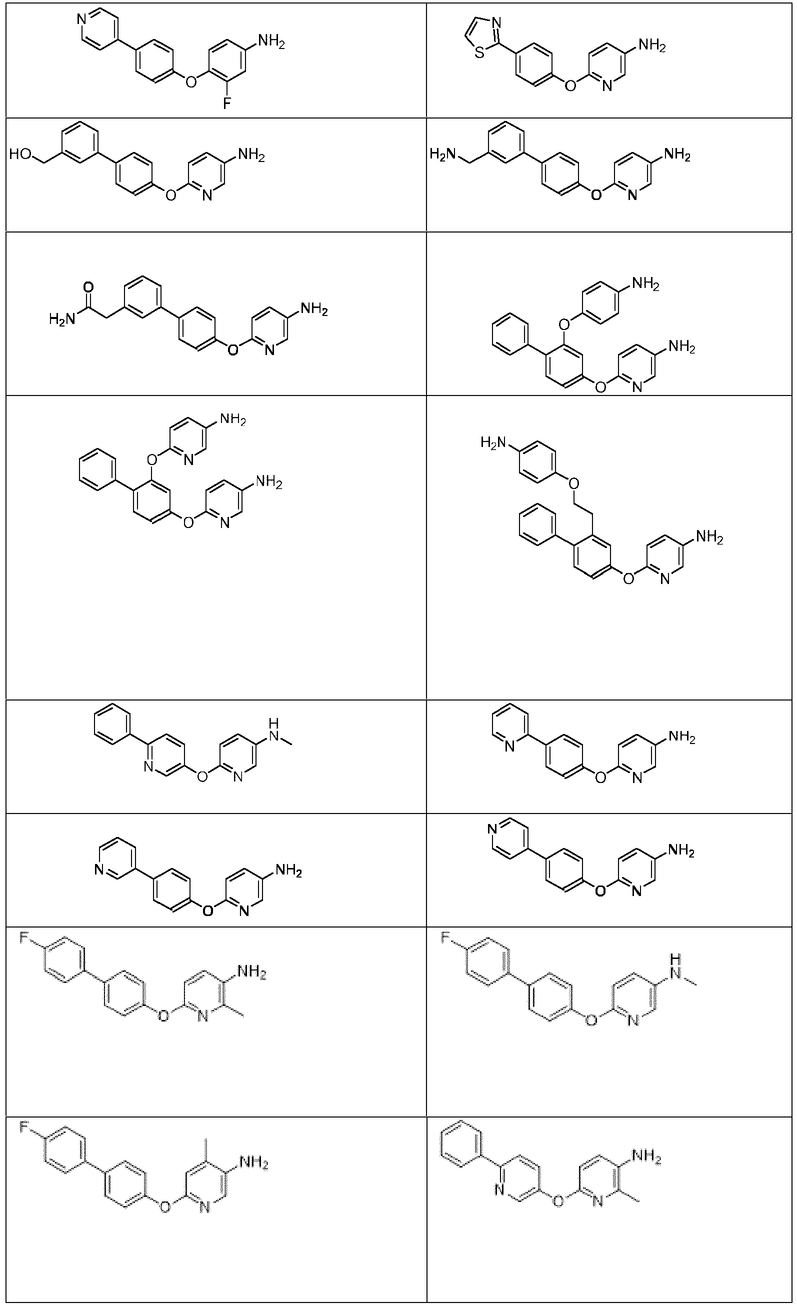

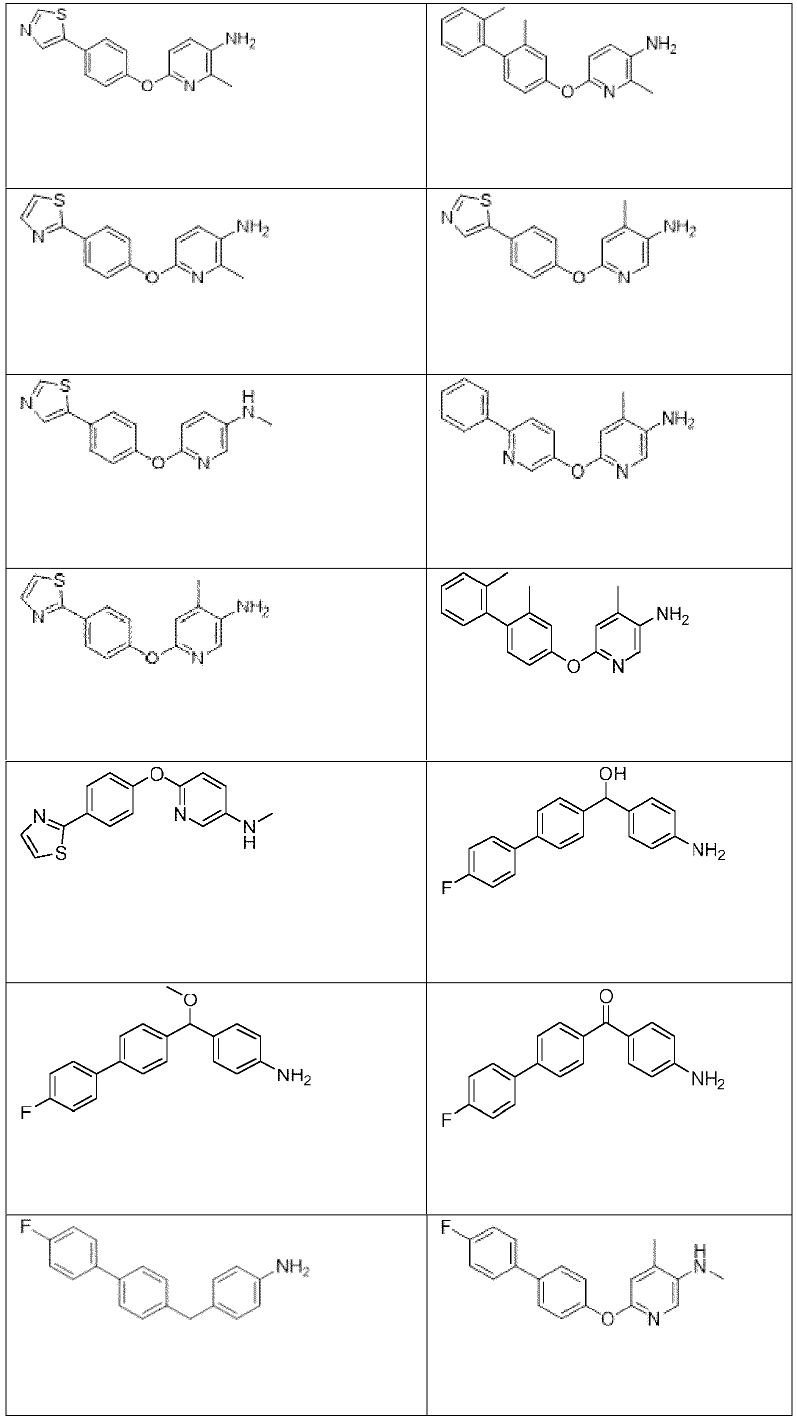

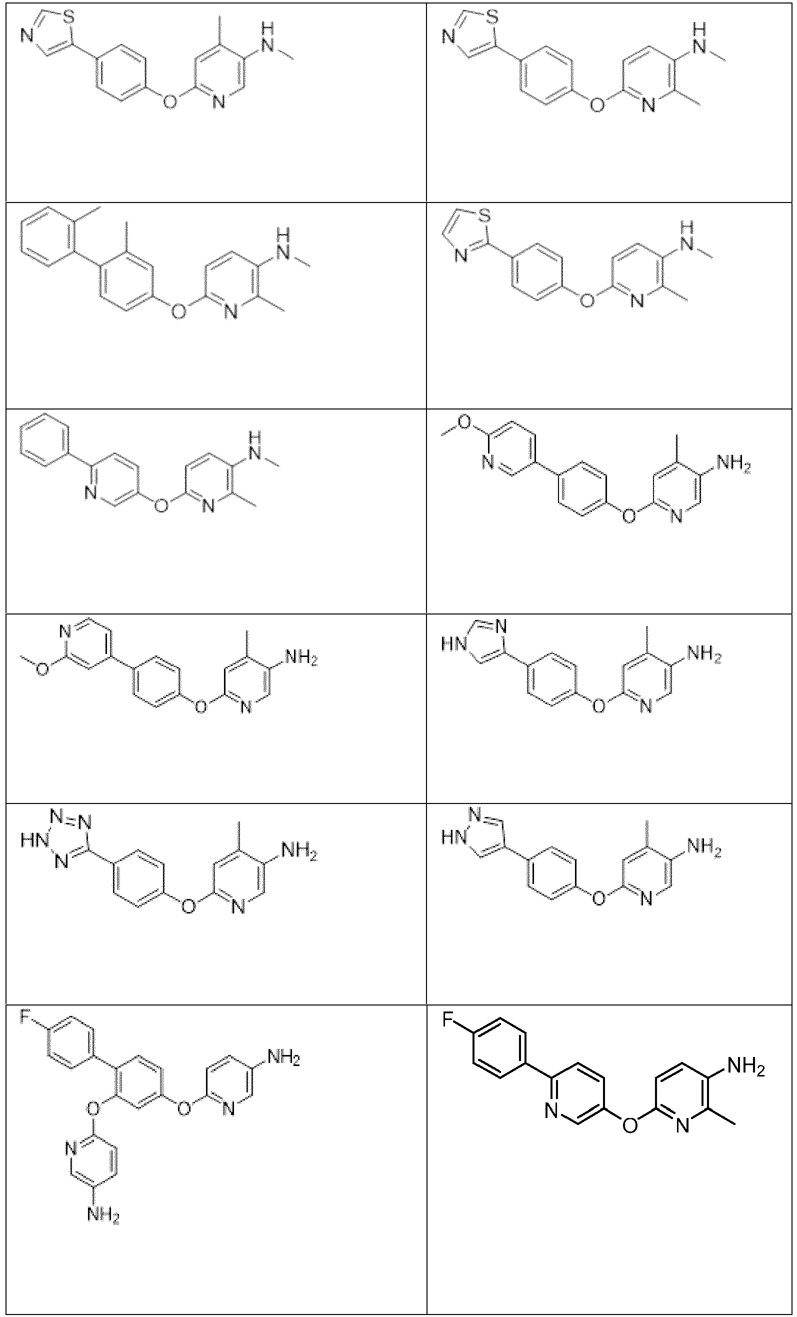

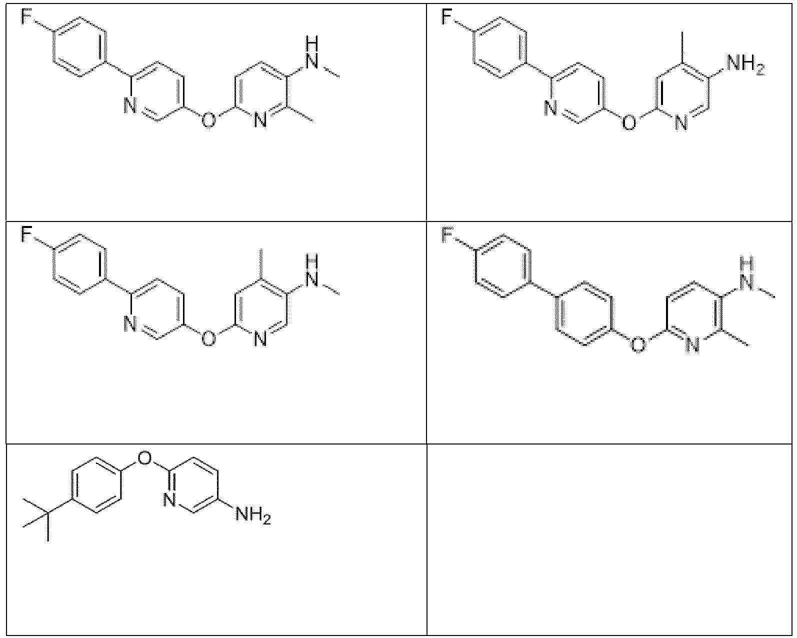

Even more particular preferred compounds of the compound of formula (I) are selected from the group consisting of

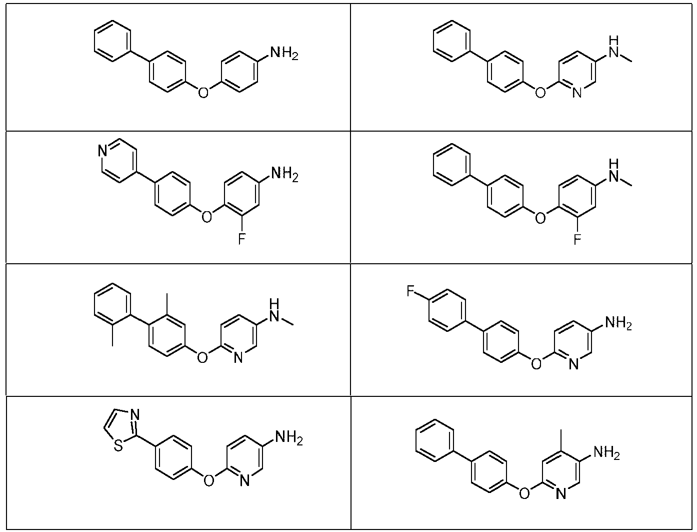

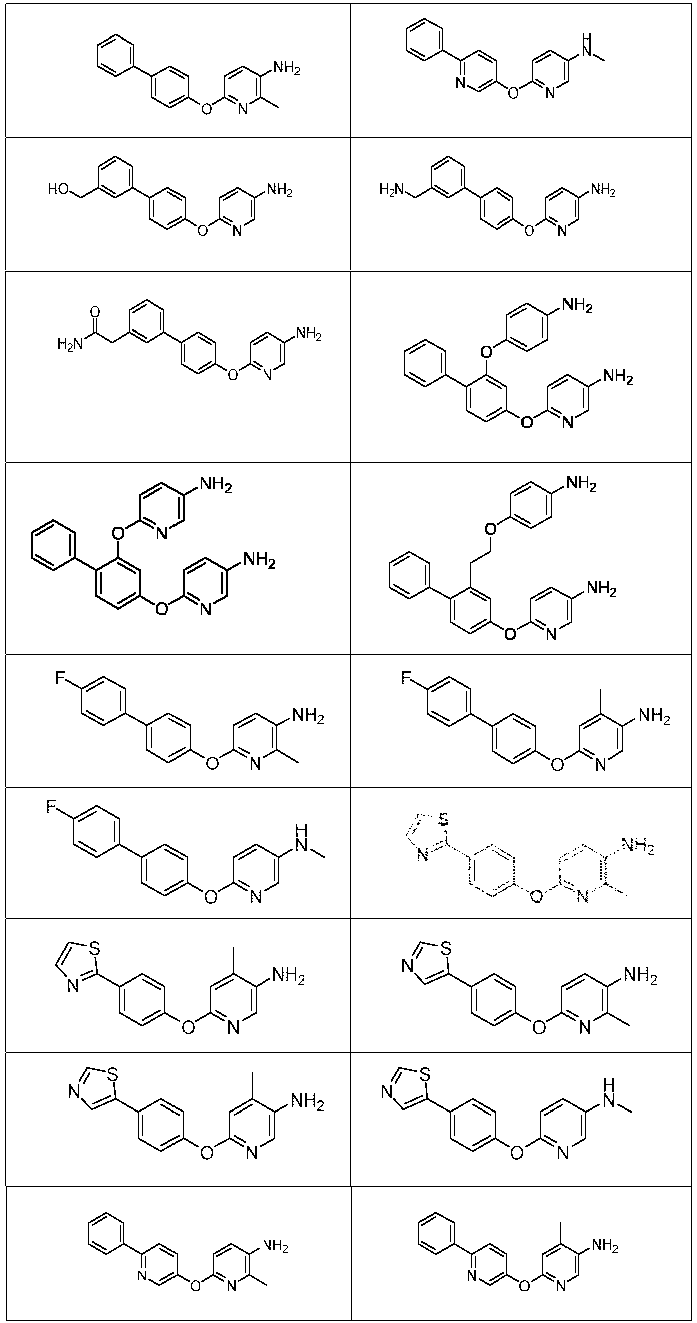

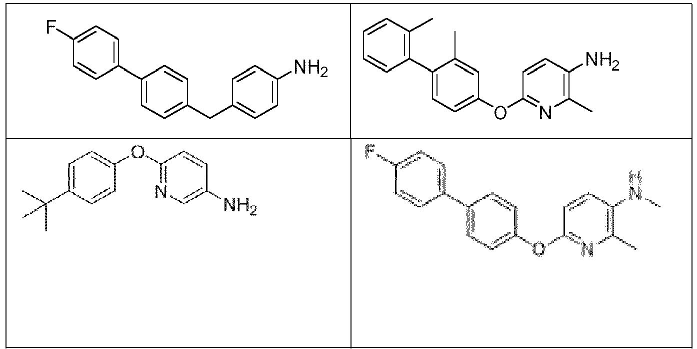

Even more particular preferred compounds of the compound of formula (I) are selected from the group consisting of

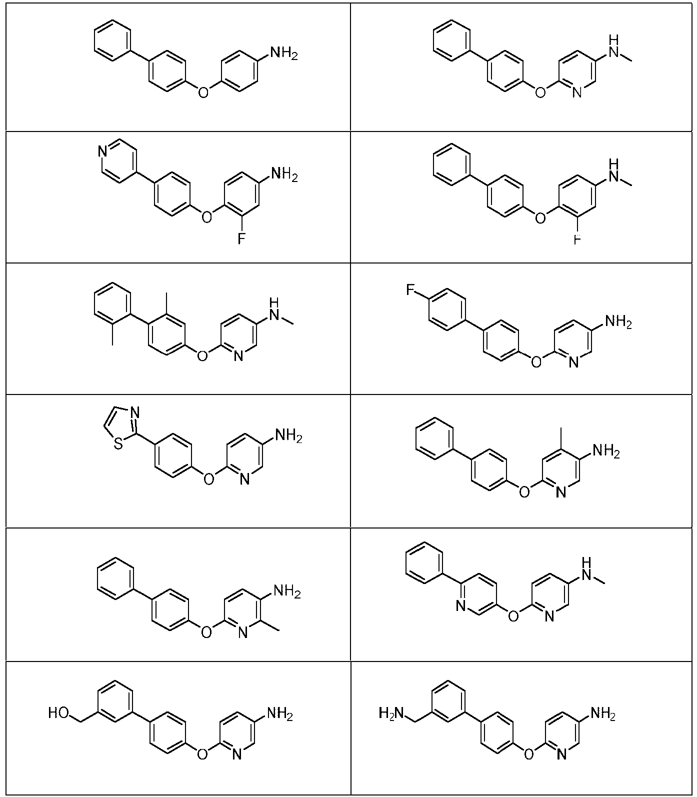

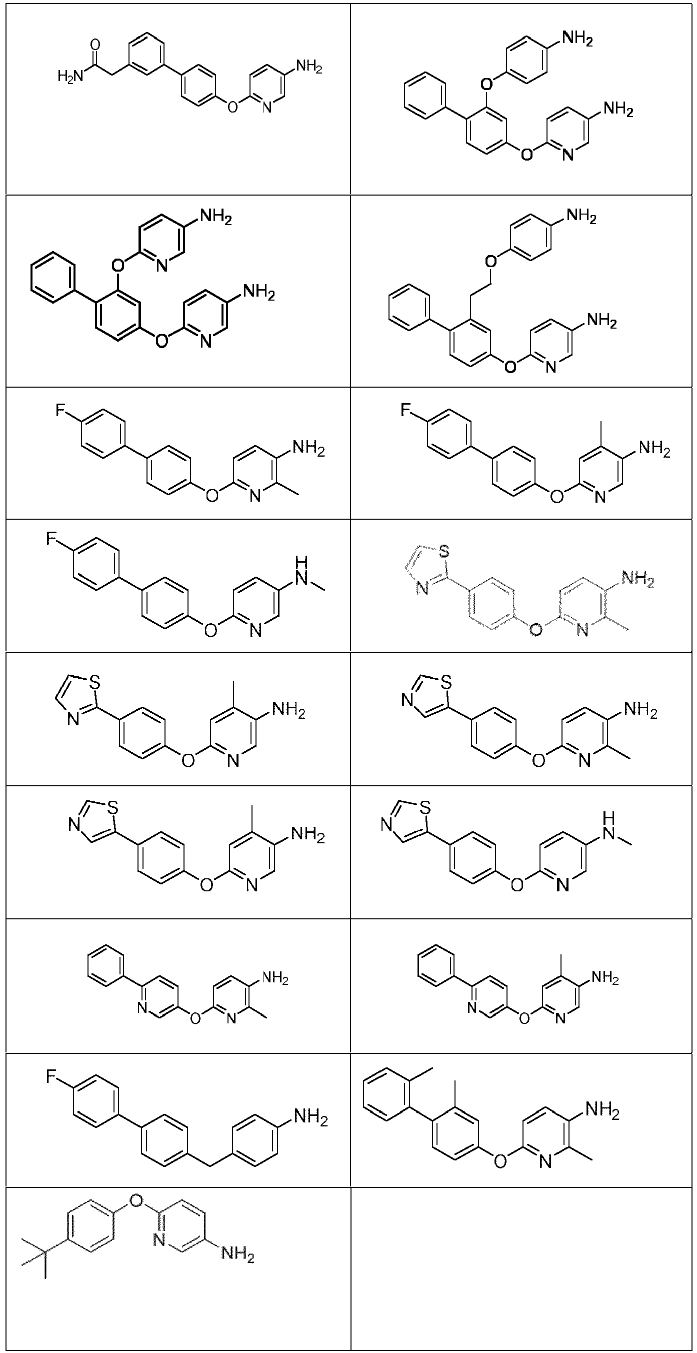

Even more particular preferred compounds of the compound of formula (I) are selected from the group consisting of

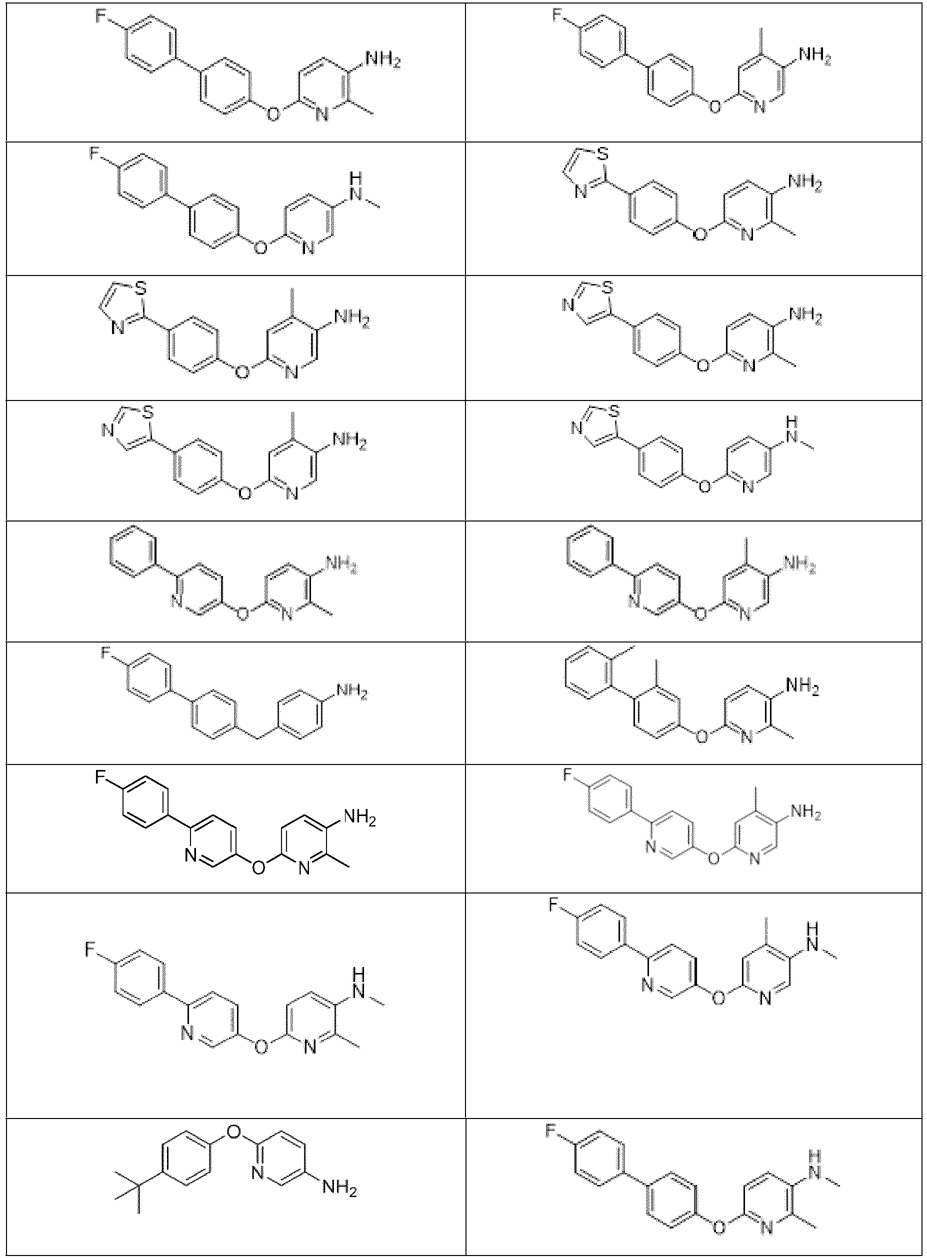

Most particular preferred compounds of the compound of formula (I) are selected from the group consisting of

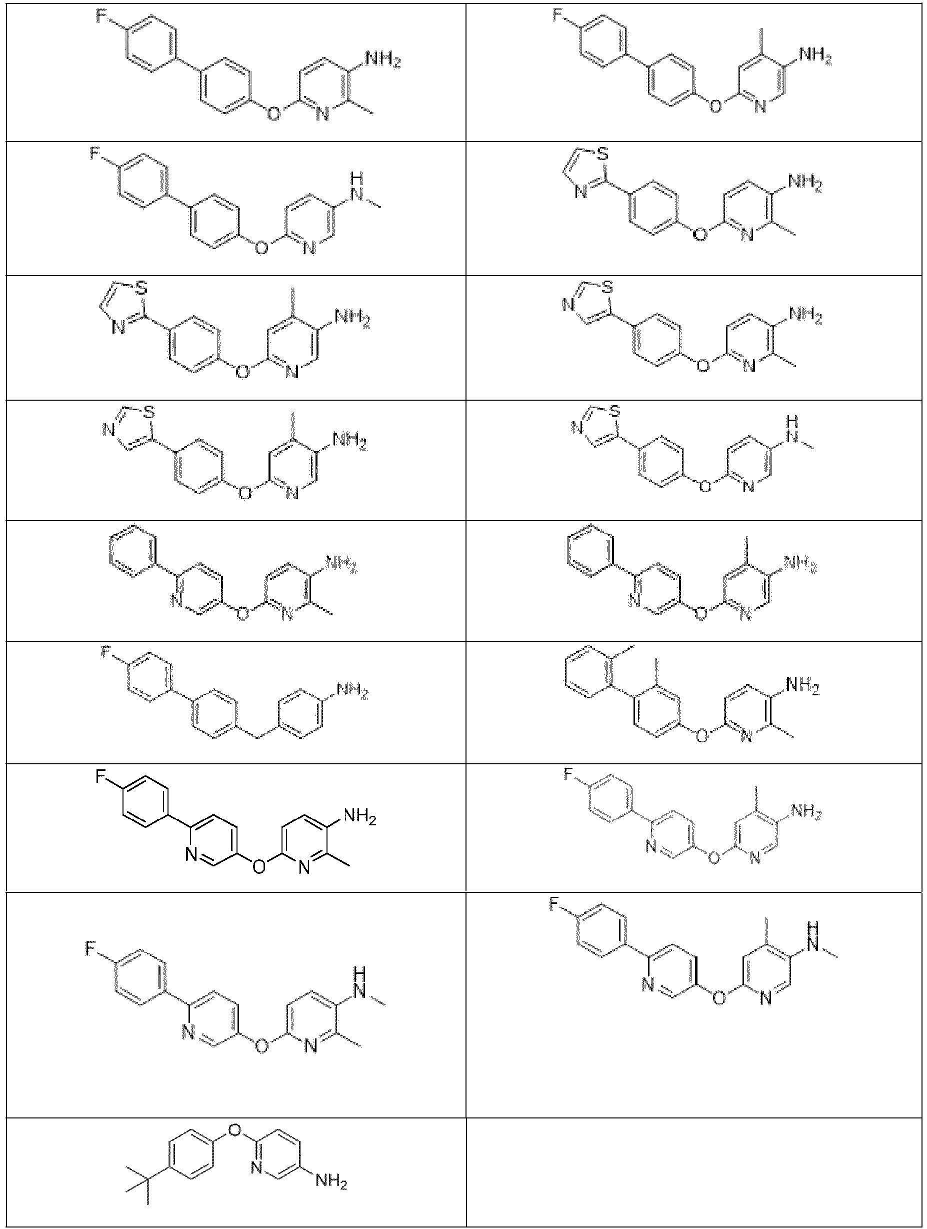

The most particular preferred T cell modulator (TCM) is selected from the group consisting of 6-((4'-fluoro-[1,1'-biphenyl]-4-yl)oxy)-2-methylpyridin-3-amine 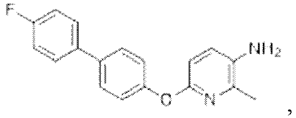 N-methyl-6-(4-(thiazol-5-yl)phenoxy)pyridin-3-amine 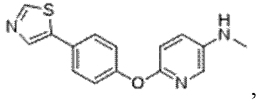 6 -(4-(tert-butylphenoxy)pyridin-3-amine 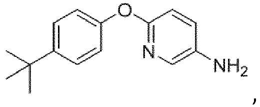 and 6-((4'-Fluoro-[1,1'-biphenyl]-4-yl)oxy)-N,2-dimethylpyridin-3-amine 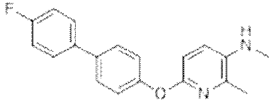 or pharmaceutically-acceptable salts, hydrates, solvates, or stereoisomers thereof.

The most preferred T cell modulator (TCM) is selected from the group consisting of 6-((4'-fluoro-[1,1'-biphenyl]-4-yl)oxy)-2-methylpyridin-3-amine 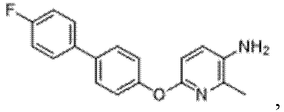 N-methyl-6-(4-(thiazol-5-yl)phenoxy)pyridin-3-amine 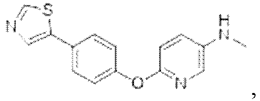 and 6 -(4-(tert-butylphenoxy)pyridin-3-amine 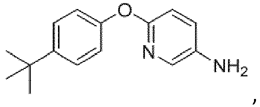 or pharmaceutically-acceptable salts, hydrates, solvates, or stereoisomers thereof, in particular 6-(4-(tert-butylphenoxy)pyridin-3-amine or pharmaceutically-acceptable salts, hydrates, solvates, or stereoisomers thereof.

### Pharmaceutical compositions

As outlined above, the invention also relates to a pharmaceutical composition comprising a T cell modulator (TCM) and optionally a pharmaceutically acceptable diluent, excipient, or carrier for use in a method for the prevention, delay of progression or treatment of an autoimmune and inflammatory disease (AIID) in a subject.

The term "pharmaceutical composition" refers to a fixed-dose combination (FDC) that includes the T cell modulator in a single dosage form, having a predetermined combination of respective dosages.

The pharmaceutical composition further may be used as add-on therapy. As used herein, "add-on" or "add-on therapy" means an assemblage of reagents for use in therapy, the subject receiving the therapy begins a first treatment regimen of one or more reagents prior to beginning a second treatment regimen of one or more different reagents in addition to the first treatment regimen, so that not all of the reagents used in the therapy are started at the same time.

The amount of the T cell modulator to be administered will vary depending upon factors such as the particular compound, disease condition and its severity, according to the particular circumstances surrounding the case, including, e.g., the specific T cell modulator being administered, the route of administration, the condition being treated, the target area being treated, and the subject or host being treated.

In one embodiment, the invention provides a pharmaceutical composition comprising a T cell modulator, wherein said T cell modulator is present in a therapeutically effective amount.

The expression "effective amount" or "therapeutically effective amount" as used herein refers to an amount capable of invoking one or more of the following effects in a subject receiving the composition of the present invention: (i) expands regulatory T cells (Treg cells) (ii) activates Treg cells (iii) inhibits proliferation of conventional T cells (iv) inhibits activation of conventional T cells (vi) inhibits activity or proliferation of cells of innate immune cells (e.g dendritic cells, macrophages etc), (vii) inhibits production or secretion of pro-inflammatory cytokines and chemokines (viii) upregulates production or enhance secretion of antiinflammatory cytokines and chemokines.

Determination of a therapeutically effective amount is well within the capability of those skilled in the art, especially in light of the detailed disclosure provided herein.

In one embodiment, the invention provides a pharmaceutical composition comprising a T cell modulator, wherein the amount of said T cell modulator in the composition is from about 1 to about 1000 mg.

### Formulations and modes of administration

A pharmaceutical composition according to the invention is, preferably, suitable for enteral administration, such as oral administration to a subject and comprises a therapeutically effective amount of the active ingredient and one or more suitable pharmaceutically acceptable diluent, excipient, or carrier.

If not indicated otherwise, a pharmaceutical composition according to the invention is prepared in a manner known per se, e.g. by means of conventional mixing, granulating, coating, dissolving or lyophilizing processes. In preparing a composition for an oral dosage form, any of the usual pharmaceutical media may be employed, for example water, glycols, oils, alcohols, carriers, such as starches, sugars, or microcrystalline cellulose, diluents, granulating agents, lubricants, binders, disintegrating agents and the like. Because of their ease of administration, tablets and capsules represent the most advantageous oral dosage unit forms, in which case solid pharmaceutical carriers are obviously employed.

In one embodiment, the pharmaceutical composition according to the invention is a composition for enteral administration. Preferred are compositions for oral administration. As indicated above, said pharmaceutical composition is preferably a pharmaceutical composition, i.e. fixed-dose composition.

A pharmaceutical composition for enteral administration is, for example, a unit dosage form, such as a tablet, a capsule or a suppository.

In one embodiment, the invention provides a pharmaceutical composition comprising a T cell modulator and at least one pharmaceutically acceptable diluent, excipient, or carrier, wherein the composition is a tablet or a capsule, preferably a tablet.

In a preferred embodiment, the invention provides a pharmaceutical composition comprising a T cell modulator and at least one pharmaceutically acceptable diluent, excipient, or carrier, wherein the composition is a sustained release tablet.

In a further preferred embodiment, the pharmaceutical composition according to the invention is for oral administration, wherein the composition is adapted to provide sustained release of the active pharmaceutical ingredients (API). Thus, the composition may increase Tₘₐₓ or reduce Cₘₐₓ, or both increase Tₘₐₓ and reduce Cₘₐₓ, as compared to an immediate release composition.

"Cₘₐₓ" means the the peak concentration of the drug in the plasma. "Tₘₐₓ" means the time from administration to reach Cₘₐₓ.

Additional or alternative, e.g. alternative materials which may be included in the composition to provide sustained release are hydrophobic polymers, for example ethyl cellulose or a methacrylic acid polymer, or a combination thereof. Such polymers, whether used singly or in combination, may be comprised in a coating or may be included in admixture with the API (i.e. may be used as a matrix-former), or may be present both in a coating and in admixture with the API.

The unit content of active ingredients in an individual dose need not in itself constitute a therapeutically effective amount, since such an amount can be reached by the administration of a plurality of dosage units. A composition according to the invention may contain, e.g., from about 10% to about 100% of the therapeutically effective amount of the active ingredients.

### Dosing regimen

An exemplary treatment regime entails administration once daily, twice daily, three times daily, every second day, twice per week, once per week. The composition of the invention is usually administered on multiple occasions. Intervals between single dosages can be, for example, less than a day, daily, every second day, twice per week, or weekly. The composition of the invention may be given as a continous uninterrupted treatment. The composition of the invention may also be given in a regime in which the subject receives cycles of treatment interrupted by a drug holiday or period of non-treatment. Thus, the composition of the invention may be administered according to the selected intervals above for a continuous period of one week or a part thereof, for two weeks, for three weeks, for four weeks, for five weeks or for six weeks and then stopped for a period of one week, or a part thereof, for two weeks, for three weeks, for four weeks, for five weeks, or for six weeks. The composition of the treatment interval and the non-treatment interval is called a cycle. The cycle may be repeated one or more times. Two or more different cycles may be used in combination for repeating the treatment one or more times. Intervals can also be irregular as indicated by measuring blood levels of said T cell modulator in the patient. In a preferred embodiment, the pharmaceutical composition according to the invention is administered once daily. In an exemplary treatment the T cell modulator can be administered from 1 - 1000 mg per day.

### Using the T cell modulator or a pharmaceutical composition thereof to treat AIID

According to an aspect the present invention provides a T cell modulator or a pharmaceutical composition thereof as described herein, for use in a method for the prevention, delay of progression or treatment of AIID in a subject.

Also provided is the use of a T cell modulator or a pharmaceutical composition thereof as described herein for the manufacture of a medicament for the prevention, delay of progression or treatment of AIID in a subject.

Also provided is the use of a T cell modulator or a pharmaceutical composition thereof as described herein for the prevention, delay of progression or treatment of AIID in a subject.

Also provided is a method for the prevention, delay of progression or treatment of AIID in a subject, comprising administering to said subject a therapeutically effective amount of a T cell modulator or a pharmaceutical composition therof as described herein.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

As used herein, "delay of progression" means increasing the time from symptoms to worsening of symptoms of AIID and includes reversing or inhibition of disease progression. "Inhibition" of disease progression or disease complication in a subject means preventing or reducing the disease progression and/or disease complication in the subject.

Preventive treatments comprise prophylactic treatments. In preventive applications, the pharmaceutical composition of the invention is administered to a subject suspected of having, or at risk for developing AIID. In therapeutic applications, the pharmaceutical composition is administered to a subject such as a patient already suffering from AIID, in an amount sufficient to cure or at least partially arrest the symptoms of the disease. Amounts effective for this use will depend on the severity and course of the disease, previous therapy, the subject's health status and response to the drugs, and the judgment of the treating physician.

In the case wherein the subject's condition does not improve, the pharmaceutical composition of the invention may be administered chronically, which is, for an extended period of time, including throughout the duration of the subject's life in order to ameliorate or otherwise control or limit the symptoms of the subject's disease or condition.

In the case wherein the subject's status does improve, the pharmaceutical composition may be administered continuously; alternatively, the dose of drugs being administered may be temporarily reduced or temporarily suspended for a certain length of time (i.e., a "drug holiday").

Once improvement of the patient's condition has occurred, a maintenance dose of the pharmaceutical composition of the invention is administered if necessary. Subsequently, the dosage or the frequency of administration, or both, is optionally reduced, as a function of the symptoms, to a level at which the improved disease is retained.

In one embodiment the autoimmune and inflammatory disease is driven by cells selected from the group consisting of Th1 cells, Th2 cells, Th17 cells and cells of innate immune system, preferably the autoimmune and inflammatory disease is driven by cells selected from the group consisting of Th1 cells, Th2 cells and Th17 cells.

In one embodiment the autoimmune and inflammatory disease is driven by Th1 cells and is selected from the group consisting of acute GvHD, chronic GvHD, Type 1 diabetes, Inflammatory bowel disease, Crohn's disease, Ulceritis, Rheumatoid arthritis, Psoriasis, Psoriatic arthritis, Multiple sclerosis and Sjogren syndrome; Th2 cells and is selected from the group consisting of Atopic dermatitis and Asthma; Th17 cells and is Psoriasis; cells of innate immune system; and/or by loss or absence Treg cells or imbalance Treg/Tconv cell ratio.

In one embodiment the autoimmune and inflammatory disease is driven by Th1 cells and is selected from the group consisting of acute GvHD, chronic GvHD, Type 1 diabetes, Inflammatory bowel disease, Crohn's disease, Ulceritis, Rheumatoid arthritis, Psoriasis, Psoriatic arthritis, Multiple sclerosis, Sjogren syndrome.

In one embodiment the autoimmune and inflammatory disease is driven by Th2 cells and is selected from the group consisting of Atopic dermatitis and Asthma.

In one embodiment the autoimmune and inflammatory disease is driven by Th17 cells and is Psoriasis.

In one embodiment the autoimmune and inflammatory disease is driven by loss or absence of Treg cells or due to imbalance of Treg/Tconv cell ratio and is selected from the group consisting of acute GvHD, chronic GvHD, steroid refractory GvHD, Type 1 diabetes, Inflammatory bowel disease, Crohn's disease, Ulceritis, Rheumatoid arthritis, Psoriasis, Psoriatic arthritis, Multiple sclerosis, Sjogren syndrome, Atopic dermatitis, Asthma, ALS.

In one embodiment the autoimmune and inflammatory disease is driven by cells of innate immune system selected from the group consisting of macrophages and dendritic cells.

In one embodiment the autoimmune and inflammatory disease is driven by a cytokine selected from the group consisting of IL-6, IFN-γ, IL-17, IL12a, IL-10, and TGF-β, or any combination thereof. In one embodiment the autoimmune and inflammatory disease is driven by IL-6. In one embodiment the autoimmune and inflammatory disease is driven by IFN-γ. In one embodiment the autoimmune and inflammatory disease is driven by IL-17. In one embodiment the autoimmune and inflammatory disease is driven by IL12a. In one embodiment the autoimmune and inflammatory disease is driven by TGF-β.

In one embodiment the autoimmune and inflammatory disease is selected from the group consisting of inflammatory diseases; autoimmune diseases; allergic diseases; autoinflammatory diseases; and Graft-versus-Host-Disease (GvHD)/transplant rejection. The term "transplant rejection" as referred herein includes organ transplants e.g. kidney transplants, liver transplants and heart transplants. In a preferred embodiment the autoimmune and inflammatory disease is selected from the group consisting of autoimmune diseases; allergic diseases; autoinflammatory diseases; and Graft-versus-Host-Disease (GvHD). In a more preferred embodiment the autoimmune and inflammatory disease is selected from the group consisting of autoimmune diseases; allergic diseases; and Graft-versus-Host-Disease (GvHD). In an even more preferred embodiment the autoimmune and inflammatory disease is selected from the group consisting of autoimmune diseases; and Graft-versus-Host-Disease (GvHD).

In a preferred embodiment the autoimmune and inflammatory disease is selected from the group consisting of acute, chronic and steroid refractory Graft-versus-Host-Disease (GvHD), Type 1 diabetes, inflammatory bowel disease, rheumatoid arthritis, Sjogren syndrome, asthma, allergies, atopic dermatitis, psoriasis, inflammation in organ transplantation, organ transplant rejection, Crohn's disease, Ulcerative colitis, Psoriatic arthritis, Systemic lupus erythematosus/Lupus nephritis, Multiple sclerosis, Neuromyelitis optica, Pemphigus vulgaris, Chronic spontaneous urticaria, Myasthenia gravis, Uveitis, autoimmune hepatitis, primary biliary cirrhosis, Behcet disease, Kawasaki disease, vasculitis, Felty syndrome, Guillain-Barre Syndrome, Autoimmune polyglandular syndrome 1, polyglandular syndrome type 2, Autoimmune pancreatitis, Celiac disease, Addison's disease, and autoimmune thyroiditis

In one embodiment the autoimmune and inflammatory disease is selected from the group consisting of acute, chronic and steroid refractory Graft-versus-Host-Disease (GvHD), Type 1 diabetes, sjogren syndrome, asthma, allergies, Ulcerative colitis, Psoriatic arthritis, Systemic lupus erythematosus/Lupus nephritis, Neuromyelitis optica, Pemphigus vulgaris, Chronic spontaneous urticaria, Myasthenia gravis, Uveitis, autoimmune hepatitis, primary biliary cirrhosis, Behcet disease, Kawasaki disease, vasculitis, Felty syndrome, Guillain-Barre Syndrome, Autoimmune polyglandular syndrome 1, polyglandular syndrome type 2, Autoimmune pancreatitis, Celiac disease, Addison's disease, and autoimmune thyroiditis.

In a more preferred embodiment the autoimmune and inflammatory disease is selected from the group consisting of acute, chronic and steroid refractory Graft-versus-Host-Disease (GvHD).

In a more preferred embodiment the autoimmune and inflammatory disease is acute GvHD.

In a more preferred embodiment the autoimmune and inflammatory disease is chronic GvHD.

In a more preferred embodiment the autoimmune and inflammatory disease is steroid refractory GvHD.

In a more preferred embodiment the autoimmune and inflammatory disease is selected from the group consisting of acute, chronic and steroid refractory Graft-versus-Host-Disease (GvHD), Ulcerative colitis, Psoriatic arthritis, Systemic lupus erythematosus/Lupus nephritis, Neuromyelitis optica, Pemphigus vulgaris, Chronic spontaneous urticaria, Myasthenia gravis, Uveitis, autoimmune hepatitis, primary biliary cirrhosis, Behcet disease, Kawasaki disease, vasculitis, Felty syndrome, Guillain-Barre Syndrome, Autoimmune polyglandular syndrome 1, polyglandular syndrome type 2, Autoimmune pancreatitis, Celiac disease, Addison's disease, and autoimmune thyroiditis.

In a more preferred embodiment the autoimmune and inflammatory disease is selected from the group consisting of Ulcerative colitis, Psoriatic arthritis, Systemic lupus erythematosus/Lupus nephritis, Neuromyelitis optica, Pemphigus vulgaris, Chronic spontaneous urticaria, Myasthenia gravis, Uveitis, autoimmune hepatitis, primary biliary cirrhosis, Behcet disease, Kawasaki disease, vasculitis, Felty syndrome, Guillain-Barre Syndrome, Autoimmune polyglandular syndrome 1, polyglandular syndrome type 2, Autoimmune pancreatitis, Celiac disease, Addison's disease, and autoimmune thyroiditis.

In a more preferred embodiment the autoimmune and inflammatory disease is Type 1 diabetes.

In an even more preferred embodiment the autoimmune and inflammatory disease is acute, chronic and steroid refractory Graft-versus-Host-Disease (GvHD), in particular acute and chronic GvHD.

In a particular preferred embodiment the autoimmune and inflammatory disease is Graft-versus-Host-Disease (GvHD) developed following allo-hematopoetic stem cell transplant (allo-HSCT).

In one embodiment Treg cells are expanded in a subject who receives a TCM compared to a control or compared to a subject who receives a vehicle. A "vehicle" as referred herein is a substance without preventive and/or therapeutic action. Thus, if a T cell modulator or a pharmaceutical composition thereof is administered to a subject according to the method of the present invention, the Treg cells of this subject or the Treg cells in an organ of the subject are expanded compared to a control or compared to cells of the subject who receives a vehicle.

In a further aspect the present invention provides a kit for use in a method for the prevention, delay of progression or treatment of an autoimmune and inflammatory disease in a subject, comprising a container and a package insert, wherein the container comprises at least one dose of a medicament comprising a T cell modulator (TCM) or a pharmaceutical composition thereof, and the package insert comprises optionally instructions for treating a subject for an autoimmune and inflammatory disease (AIID) using the medicament.

In a further aspect the present invention provides a T cell modulator (TCM) for use in a method for the prevention, delay of progression or treatment of an autoimmune and inflammatory disease (AIID) in a subject in combination with standard of care or immunosuppressive drugs. "Standard of care" as refered herein describes a treatment process approved in clinics for a certain disease and includes treatment with Ruxolitinib, Tocilizumab, Infliximab, Secukinumab, Dupilumab. Immunosuppressive drugs as refered herein includes different types of compounds which have the effect of dampening the response of the immune system. Corticosteroids are an example of immunosuppressive drugs.

### Examples

The present examples are intended to illustrate the present invention.

### Material and Methods:

**Human PBMCs and mouse splenocyte *in vitro* stimulation and treatment:** Human peripheral blood mononuclear cells (PBMCs) and mouse splenocytes from donors were stimulated *in vitro* with anti-CD3 and anti-CD28 antibodies. The stimulated cells were simultaneously treated with compounds for 96 hours. The T cells were profiled for different cell surface and intracellular markers and proliferation by flow cytometry.

***In vivo* treatment of mice and pharmacodynamic response:** Mice experiments were performed in compliance with authorization to perform animal experiments (VD3323). Briefly, 6-8 weeks old wild type C57Bl6 mice were subcutaneously or intraperitoneally dosed with vehicle or compounds for different durations. Mice were sacrificed, spleens were harvested 24 hours post-last dosing. Splenocytes were analyzed for cell surface and intracellular markers expression using flow cytometry. RNA expression analyses were performed using quantitative real time PCR.

**Humanized GvHD mouse model:** 6-8 weeks old NSG mice were intraperitoneally injected with 10 million human peripheral blood mononuclear cells (PBMCs) as below:
***Ex vivo* treatment of PBMCs:** 10 million human PBMCs from healthy donor #1 were *ex-vivo* treated with DMSO and 10 mM of 6-(4-tert-butylphenoxy)pyridin-3-amine for 6 hours. Ex-vivo treated PBMCs were intraperitoneally injected into mice (10 million per mouse). 10 NSG mice were injected with DMSO pre-treated PBMCs and 10 NSG mice were injected with 6-(4-tert-butylphenoxy)pyridin-3-amine pre-treated PBMCS. Following PBMC injections mice were monitored for up to 42 days for symptoms of GvHD development as measured by standardized GvHD clinical score system. No *in-vivo* drug treatment was administered.

Animals were scored according to GvHD clinical scoring system as shown in table 1 below:

**Table 1: GvHD clinical score**

| | SCORE (point) | | |
|---|---|---|---|
| **Criteria** | **0** | **1** | **2** |
| Body weight loss (%) | <5% | 5-15% | >15% |
| Posture | normal | hunching at rest | severe hunching impairs movements |
| Activity | normal | mild-moderately decreased | immobile unless stimulated |
| Fur texture | normal | mild-moderate ruffling | severe ruffling and grooming |
| Skin Integrity | normal | scaling of paw and tail | obvious areas of denuded skin |
| Paleness | normal | slight | severe |

A cumulative GvHD clinical score was calculated by addition of scores for individual parameters (e.g cumulative score = BWL + posture + activity+ fur texture + skin integrity + paleness).

***Ex-vivo* pre-treatment of PBMCs and *in-vivo* treatment of mice:** Human PBMCs were *ex-vivo* treated with either DMSO or 6-(4-tert-butylphenoxy)pyridin-3-amine and then intraperitoneally injected into NSG mice. Mice were *in vivo* treated with different compounds as below:
i. DMSO *ex vivo* hPBMCs treatment (7 hrs), plus vehicle *in vivo* treatment
ii. DMSO *ex vivo* hPBMCs treatment (7 hrs), plus 6-(4-tert-butylphenoxy)pyridin-3-amine *in vivo* treatment for 3 days
iii. 6-(4-tert-butylphenoxy)pyridin-3-amine *ex vivo* hPBMCs treatment (7 hrs), plus 6-(4-tert-butylphenoxy)pyridin-3-amine *in vivo* treatment for 3 days
iv. DMSO *ex vivo* PBMCs treatment (7 hrs) plus Ruxolitinib *in vivo* treatment for 3 days
v. 6-(4-tert-butylphenoxy)pyridin-3-amine *ex vivo* hPBMCs treatment (7 hrs), plus Ruxolitinib *in vivo* treatment for 3 days

Following PBMC injections, mice were treated with vehicle or 6-(4-tert-butylphenoxy)pyridin-3-amine or Ruxolitinib starting at day 0. Mice were monitored from 60-85 days for symptoms of GvHD and scored according to a standard clinical scoring system for GvHD (Naserian et al., 2018) (see table 1) and a cumulative clinical score was obtained by addition of clinical score for individual parameters. A maximum score of 6 is measured when reaching endpoint.

**Cell proliferation assay:** The effect of compounds on T cell proliferation was quantified using Cell trace violet (CTV). PBMCs/splenocytes were incubated with CTV for 20 minutes at 37°C. Cells were washed twice with RPMI complete medium. CTV labelled cells were stimulated with anti-CD3 and anti-CD28 antibodies and treated with control or compounds and cultured at 37°C for 96 hours. The cell division index was measured by flow cytometry.

**Flow cytometry analysis:** Flow cytometry analysis was performed to quantify cell surface and intracellular markers expression and measure cell division index.

**Viability staining:** Cells were transferred into a 96 well conical bottom plate and washed with serum free PBS1x. Cells were incubated with Zombie NIR (Biolegend #423105) + FC block (CD16/31) for 20 minutes at 4°C. Cells were washed with staining medium (SM) (1x HBSS, 2.5mM EDTA, 2% NBS

**Cell surface marker staining:** Cells were stained with antibodies against cell surface markers (CD3, CD4, CD8, CD25, Biolegend or ThermoFisher) for 20 minutes at 4°C. Cells were washed with SM before continuing with nuclear staining.

**FOXP3 nuclear staining:** Cells were fixed and permeabilized with Foxp3 Transcription Factor Staining Buffer Set (ThermoFisher #00-5523) for 20 minutes at room temperature. Following washing with 1x permeabilization buffer, cells were incubated with blocking solution for 15 minutes at room temperature. The appropriate dilution of FOXP3 antibody (Biolegend or ThermoFisher) was the added to the blocking solution. Cells were then resuspended and incubated for 30 minutes at room temperature. Following washing with 1x permeabilization buffer, cells were resuspended in staining media for FACS acquisition by Cytoflex S (Beckman Coulter). Results are finally analysed with the software FlowJo v.10.7.1.

**ELISA assay:** Supernatant from activated human PBMC cultures described above was separated from cells at the end of culture time. Key cytokines (IL-6, IFN-γ, IL-17) were quantified using sandwich ELISA reagents set (Diaclone) following manufacturer's instruction manual.

**Cytokine profiling in human plasma samples:** Plasma samples from patients treated with 6-(4-tert-butylphenoxy)pyridin-3-amine were collected and stored at -80 °C until analysed. Cytokine and chemokine profiling was performed using MesoScale Discovery (MSD) platform and three assay panels: 1) Pro-inflammatory panel human, 2) Human IL-6 assay S-Plex, 3) Human IL-10 assay S-Plex.

**Allogeneic Bone marrow transplant:** Recipient Balb/c mice were irradiated twice 3.75Gy (T=0 and T=4h) in an X-ray source irradiator before transplantation at T=8h. Treatment with antibiotics (Nopil) and analgesic (Dafalgan) was supplemented to drinking water 4 days before injection. Irradiated recipient Balb/c mice were transplanted with 5 million T cell depleted bone marrow+ 1 million splenocytes. Allogeneic recipient animals were treated with vehicle or 6-(4-tert-butylphenoxy)pyridin-3-amine subcutaneously at 20 mg/kg for two days immediately after allo-BM transplant, given a 10 days treatment holiday to recover body weight loss due to whole body irradiation and then treatment continued at 20 mg/kg . A last group was treated intraperitoneally with Cyclosporin A at 5 mg/kg for 2 days (QD), and then treatment continued at 5 mg/kg (QDX5) after break for 10 days. Animals were monitored for symptoms of GvHD development (severe body weight loss, alopecia and diarrhea.

**Cytokine analysis in allogeneic Bone marrow transplant:** The recipient animals were bled on day 36 and blood plasma was extracted to determine inflammatory cytokine concentration (IFNγ, IL-6, IL-17a) by LEGENDplexTM multiplexed assay.

### Results:

### Expansion of Treg cells

As can be seen from Table 2 below and Figures 1 and 2, respectively, compounds lead to an expansion of Treg cells in activated mouse splenocytes and in vivo treated mice.

**Table 2: Compounds leads to an expansion of Treg cells in activated human PBMC cultures.**

| **Control (untreated)** | **6-(4-tert-butylphenoxy)pyridin-3-amine** | **6-((4'-fluoro-[1,1'-biphenyl]-4-yl)oxy)-2-methylpyridin-3-amine** |
|---|---|---|
| 0% | 112% | 120% |

### Inhibition of proliferation of activated CD4 T cells

As can be seen from Table 3 below compounds inhibit proliferation of activated CD4 T cells.

**Table 3: Compounds inhibit proliferation of activated CD4 T cells: Percentage inhibition of CD4 T cells.**

| **Control** | **6-(4-tert-butylphenoxy)pyridin-3-amine** | **6-((4'-fluoro-[1,1'-biphenyl]-4-yl)oxy)-2-methylpyridin-3-amine** |
|---|---|---|
| 0 % | 81 % | 40 % |

### Inhibition of proliferation of activated CD8 T cells

As can be seen from Table 4 below compounds inhibit proliferation of activated CD8 T cells.

**Table 4: Compounds inhibit proliferation of activated CD8 T cells. Percentage inhibition of CD8 T cells.**

| **Control** | **6-(4-tert-butylphenoxy)pyridin-3-amine** | **6-((4'-fluoro-[1,1'-biphenyl]-4-yl)oxy)-2-methylpyridin-3-amine** |
|---|---|---|
| 0 % | 56 % | 74 % |

### Inhibition of pro-inflammatory cytokine expression

As can be seen from Table 5 below compounds inhibit pro-inflammatory cytokine expression.

**Table 5: Compounds inhibit pro-inflammatory cytokine expression. Percentage downregulation of pro-inflammatory cytokines in activated human PBMC cultures.**

| **Cytokine** | **Control** | **6-(4-tert-butylphenoxy)pyridin-3-amine** | **6-((4'-fluoro-[1,1'-biphenyl]-4-yl)oxy)-2-methylpyridin-3-amine** | **N-methyl-6-(4-(thiazol-5-yl)phenoxy)pyridin-3-amine** |
|---|---|---|---|---|
| **IL-6** | 0 % | 61 % | 66 % | 80 % |
| **IL-17** | 0 % | 38% | 78 % | 85 % |
| **IFNG** | 0 % | 10 % | 41 % | 38 % |

### Inhibition of pro-inflammatory cytokine expression

As can be seen from Table 6 below 6-(4-tert-butylphenoxy)pyridin-3-amine inhibits pro-inflammatory cytokines in human patients.

**Table 6: 6-(4-tert-butylphenoxy)pyridin-3-amine inhibits pro-inflammatory cytokines in human patients. Percentage downregulation of pro-inflammatory cytokines in blood plasma of human patients treated with 6-(4-tert-butylphenoxy)pyridin-3-amine.**

| **Cytokine** | **At baseline pre-treatment** | **6-(4-tert-butylphenoxy)pyridin-3-amine** |
|---|---|---|
| IL-6 | 0 % | 57 % (day 3 on treatment) |
| IFNG | 0 % | 90 % (day 29 on treatment) |

### Upregulation of anti- inflammatory cytokine

As can be seen from Table 7 below and Figures 3 and 4, respectively, 6-(4-tert-butylphenoxy)pyridin-3-amine upregulates anti- inflammatory cytokine in human patients.

**Table 7: 6-(4-tert-butylphenoxy)pyridin-3-amine upregulates anti- inflammatory cytokine in human patients. Percentage upregulation of IL-10 in blood plasma of human patient treated with 6-(4-tert-butylphenoxy)pyridin-3-amine.**

| **Cytokine** | **At baseline** | **6-(4-tert-butylphenoxy)pyridin-3-amine** |
|---|---|---|
| IL-10 | 0 % | 60 % |

### Prophylactic treatment with 6-(4-tert-butylphenoxy)pyridin-3-amine treats humanized mouse model of GvHD:

As can be seen from Table 8 below treatment with 6-(4-tert-butylphenoxy)pyridin-3-amine decreased cumulative GvHD score significantly in animals injected with human PBMCs pre-treated (ex-vivo) with DMSO and 6-(4-tert-butylphenoxy)pyridin-3-amine.

**Table 8: GvHD clinical score at day 21 in animals injected with human PBMCs pre-treated (ex-vivo) with DMSO and 6-(4-tert-butylphenoxy)pyridin-3-amine.**

| **GvHD clinical score at day 21** | **DMSO treatment** | **6-(4-tert-butylphenoxy)pyridin-3-amine treatment** |
|---|---|---|
| **Body weight loss** | 1.0 | 0 |
| **Paleness** | 0.71 | 0 |
| **Posture** | 0.71 | 0.14 |
| **Skin integrity** | 0.14 | 0 |
| **Activity score** | 0.71 | 0.14 |
| **Fur texture** | 0.57 | 0.14 |
| **Cumulative GvHD score** | 3.84 | 0.42 |

As can be seen from Table 9 below treatment with 6-(4-tert-butylphenoxy)pyridin-3-amine decreased cumulative GvHD score significantly in NSG mice injected with ex-vivo treated human PBMCs followed by in vivo treatment with 6-(4-tert-butylphenoxy)pyridin-3-amine and Ruxolitinib.

**Table 9: GvHD clinical score in NSG mice injected with ex-vivo treated human PBMCs followed by in vivo treatment with 6-(4-tert-butylphenoxy)pyridin-3-amine and Ruxolitinib.**

| **GvHD clinical score** | **DMSO/Vehicle** | **DMSO/6-(4-tert-butylphenoxy )pyridin-3-amine** | **6-(4-tert-butylphen oxy)pyridin -3-amine /6-(4-tert-butylphen oxy)pyridi n-3-amine** | **DMSO/Ru xolitinib** | **6-(4-tert-butylphenoxy) pyridin-3-amine /Ruxolitinib** |
|---|---|---|---|---|---|
| **Body weight loss (D59)** | 2.0 | 1.35 | 0.9 | 1.6 | 0.9 |
| **Paleness (D59)** | 0.9 | 1.2 | 1.2 | 0.9 | 1.8 |
| **Posture (D59)** | 1.3 | 1.3 | 0.7 | 1.2 | 1.1 |
| **Skin integrity (day 45)** | 1.25 | 0.65 | 0.7 | 1.2 | 0.6 |
| **Activity score** | 1.0 | 0.5 | 0.5 | 0.9 | 0.8 |
| **Fur texture (day 45)** | 1.35 | 0.95 | 1.2 | 1.1 | 1.0 |
| **Cumulative GvHD score** | 7.8 | 5.95 | 5.2 | 6.9 | 6.2 |

### Alleviation of GvHD in an allo-bone marrow transplant model:

Graft vs host disease (GvHD) is an example of alloimmune disease that develops following allo-hematopoetic stem cell transplant (allo-HSCT) in humans. The activity of 6-(4-tert-butylphenoxy)pyridin-3-amine and a calcineurin inhibitor Cyclosprin A was investigated in a mouse model of GvHD following allo-bone marrow transplant. In this model, T cell depleted bone marrow cells (TCD-BM) + splenocytes from C57Bl6 mice were transplanted into irradiated Balb/c mice. As shown in figure 5, while 60 % of vehicle treated animals died due to GvHD, only 20 % of 6-(4-tert-butylphenoxy)pyridin-3-amine treated developed GvHD. Cyclosporin A, which is used as standard of care for the prevention of GvHD did not perform better than vehicle treated animals. This data shows that in an allogeneic-hematopoetic stem cell transplanted (or allo-bone marrow transplant) setting, 6-(4-tert-butylphenoxy)pyridin-3-amine prevents development of GvHD. In addition, treatment of allo-BM + splenocyte transplanted mice with 6-(4-tert-butylphenoxy)pyridin-3-amine led to a downregulation of cytokines implicated in GvHD pathophysiology (INFG, IL-17 and IL-6, figure 6) and an expansion of regulatory T cells.

## Claims

1. A T cell modulator (TCM) for use in a method for the prevention, delay of progression or treatment of an autoimmune and inflammatory disease (AIID) in a subject, wherein the T cell modulator (TCM) is an inhibitor of NOTCH transcription complex, and wherein the T cell modulator (TCM) is a compound of formula (I) 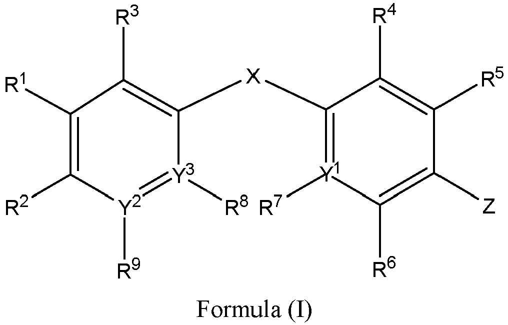
pharmaceutically-acceptable salts, hydrates, solvates, or stereoisomers thereof,
wherein X is selected from CH₂, CF₂, CHF, CO, CHOH, CHO(C₁-C₃) alkyl, NH, N(C₁-C₃ alkyl), S, SO and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl, C₁-C₆ heteroalkyl, C₃-C₁₂ cycloalkyl, C₃-C₁₂ heterocyclyl and C₁-C₆ alkoxy;
wherein R⁴, R⁵ and R⁶ are each independently selected from H, halogen, CN, C₁-C₆ alkoxy, C₁-C₆-S-alkyl, C₁-C₆ alkyl, C₁-C₆ heteroalkyl, C₃-C₁₂ cycloalkyl and C₃-C₁₂ heterocyclyl;
wherein R⁷ is absent when Y¹ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y¹ is C;
wherein R⁸ is absent when Y³ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y³ is C;
wherein R⁹ is absent when Y² is N or is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R¹² is selected from H, NH₂, NHC₁-C₆ alkyl, C₁-C₆ alkyl, C₁-C₆ heteroalkyl, C₂-C₆ alkenyl, C₂-C₆ alkynyl, C₃-C₁₂ cycloalkyl and C₃-C₁₂ heterocyclyl; and optionally one or more pharmaceutically acceptable diluents, excipients or carriers; with the proviso that if R² is C₁-C₆ alkyl or C₃-C₁₂ cycloalkyl, Y¹ is N.

2. The T cell modulator (TCM) for use according to claim 1, wherein the T cell modulator (TCM) is selected from the group consisting of 6-((4'-fluoro-[1,1'-biphenyl]-4-yl)oxy)-2-methylpyridin-3-amine, N-methyl-6-(4-(thiazol-5-yl)phenoxy)pyridin-3-amine, and 6-(4-(tert-butylphenoxy)pyridin-3-amine or pharmaceutically-acceptable salts, hydrates, solvates, or stereoisomers thereof.

3. The T cell modulator (TCM) for use according to claim 1, wherein the T cell modulator (TCM) is 6-(4-(tert-butylphenoxy)pyridin-3-amine or pharmaceutically-acceptable salts, hydrates, solvates, or stereoisomers thereof.

4. The T cell modulator (TCM) for use according to any one of claims 1-3, wherein the autoimmune and inflammatory disease is selected from the group consisting of acute, chronic and steroid refractory Graft-versus-Host-Disease (GvHD), Type 1 diabetes, inflammatory bowel disease, rheumatoid arthritis, Sjogren syndrome, asthma, allergies, atopic dermatitis, psoriasis, inflammation in organ transplantation, organ transplant rejection, Crohn's disease, Ulcerative colitis, Psoriatic arthritis, Systemic lupus erythematosus/Lupus nephritis, Multiple sclerosis, Neuromyelitis optica, Pemphigus vulgaris, Chronic spontaneous urticaria, Myasthenia gravis, Uveitis, autoimmune hepatitis, primary biliary cirrhosis, Behcet disease, Kawasaki disease, vasculitis, Felty syndrome, Guillain-Barre Syndrome, Autoimmune polyglandular syndrome 1, polyglandular syndrome type 2, Autoimmune pancreatitis, Celiac disease, Addison's disease, and autoimmune thyroiditis.

5. The T cell modulator (TCM) for use according to any one of claims 1-3, wherein the autoimmune and inflammatory disease is driven by Th1 cells and is selected from the group consisting of acute GvHD, chronic GvHD, Type 1 diabetes, Inflammatory bowel disease, Crohn's disease, Ulceritis, Rheumatoid arthritis, Psoriasis, Psoriatic arthritis, Multiple sclerosis, Sjogren syndrome.

6. The T cell modulator (TCM) for use according to any one of claims 1-3, wherein the autoimmune and inflammatory disease is is selected from the group consisting of Atopic dermatitis and Asthma or wherein the autoimmune and inflammatory disease is driven by Th17 cells and is Psoriasis.

7. The T cell modulator (TCM) for use according to any one of claims 1-3, wherein the autoimmune and inflammatory disease is driven by cells of innate immune system selected from the group consisting of macrophages and dendritic cells.

8. The T cell modulator (TCM) for use according to any one of claims 1-3, wherein the autoimmune and inflammatory disease is Graft-versus-Host-Disease (GvHD).

9. The T cell modulator (TCM) for use according to any one of claims 1-3, wherein the autoimmune and inflammatory disease is acute, chronic and steroid refractory Graft-versus-Host-Disease (GvHD).

10. A pharmaceutical composition for use in a method for the prevention, delay of progression or treatment of an autoimmune and inflammatory disease comprising the T cell modulator (TCM) according to any one of claims 1-3 and optionally one or more pharmaceutically acceptable diluents, excipients or carriers.

11. A kit for use in a method for the prevention, delay of progression or treatment of an autoimmune and inflammatory disease in a subject, comprising comprising a container and a package insert, wherein the container comprises at least one dose of a medicament comprising the T cell modulator (TCM) according to any one of claims 1-3 or the pharmaceutical composition of claim 10, and the package insert comprises optionally instructions for treating a subject for an autoimmune and inflammatory disease (AIID) using the medicament.

## Patentansprüche

1. T-Zell-Modulator (TCM) zur Verwendung bei einem Verfahren zur Vorbeugung, Verzögerung des Fortschreitens oder Behandlung einer Autoimmun- und Entzündungskrankheit (Autoimmune and Inflammatory Disease, AIID) bei einem Individuum, wobei der T-Zell-Modulator (TCM) ein Inhibitor eines NOTCH-Transkriptionskomplexes ist und wobei es sich bei dem T-Zell-Modulator (TCM) um eine Verbindung der Formel (I), 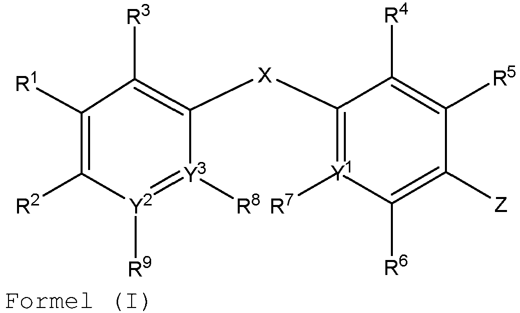
pharmazeutisch unbedenkliche Salze, Hydrate, Solvate oder Stereoisomere davon handelt,
wobei X aus CH₂, CF₂, CHF, CO, CHOH, CHO(C₁-C₃)-Alkyl, NH, N(C₁-C₃-Alkyl), S, SO und O ausgewählt ist;
wobei Y¹, Y² und Y³ jeweils unabhängig aus N und C ausgewählt sind;
wobei Z für NR¹⁰R¹¹ steht, wobei R¹⁰ und R¹¹ jeweils unabhängig aus H und C₁-C₆-Alkyl ausgewählt sind;
wobei R¹ aus H, Halogen, C₁-C₆-Alkyl, C₃-C₁₂-Cycloalkyl, C₃-C₁₂-Heterocyclyl, C₁-C₆-Alkoxy, C₁-C₆-Heteroalkyl, C₀-C₃-AlkylOC₀-C₃-alkylaryl, wobei das Aryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert ist; C₀-C₃-AlkylOC₀-C₃-alkylheteroaryl, wobei das Heteroaryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert ist; und C₁-C₆-Alkyl, das durch Aryl oder Heteroaryl substituiert ist, wobei das Aryl und das Heteroaryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert sind, ausgewählt ist;
wobei R² aus C₁-C₆-Alkyl, C₃-C₁₂-Cycloalkyl, Aryl und Heteroaryl ausgewählt ist, wobei das Aryl und das Heteroaryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl, C(O)R¹², C₁-C₆-Alkyl-C(O)R¹² substituiert sind;
wobei R³ aus H, Halogen, C₁-C₆-Alkyl, C₁-C₆-Heteroalkyl, C₃-C₁₂-Cycloalkyl, C₃-C₁₂-Heterocyclyl und C₁-C₆-Alkoxy ausgewählt ist;
wobei R⁴, R⁵ und R⁶ jeweils unabhängig aus H, Halogen, CN, C₁-C₆-Alkoxy, C₁-C₆-S-Alkyl, C₁-C₆-Alkyl, C₁-C₆-Heteroalkyl, C₃-C₁₂-Cycloalkyl und C₃-C₁₂-Heterocyclyl ausgewählt sind;
wobei R⁷ fehlt, wenn Y¹ N ist, oder aus H, Halogen, C₁-C₆-Alkyl, C₁-C₆-Heteroalkyl, C₃-C₁₂-Cycloalkyl, C₃-C₁₂-Heterocyclyl und C₁-C₆-Alkoxy ausgewählt ist, wenn Y¹ C ist;
wobei R⁸ fehlt, wenn Y³ N ist, oder aus H, Halogen, C₁-C₆-Alkyl, C₁-C₆-Heteroalkyl, C₃-C₁₂-Cycloalkyl, C₃-C₁₂-Heterocyclyl und C₁-C₆-Alkoxy ausgewählt ist, wenn Y³ C ist;
wobei R⁹ fehlt, wenn Y² N ist, oder aus H, Halogen, C₁-C₆-Alkyl, C₃-C₁₂-Cycloalkyl, C₃-C₁₂-Heterocyclyl, C₁-C₆-Alkoxy, C₁-C₆-Heteroalkyl, C₀-C₃-AlkylOC₀-C₃-alkylaryl, wobei das Aryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert ist; C₀-C₃-AlkylOC₀-C₃-Alkylheteroaryl, wobei das Heteroaryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert ist, und C₁-C₆-Alkyl, das durch Aryl oder Heteroaryl substituiert ist, wobei das Aryl und das Heteroaryl gegebenenfalls durch NH₂, OC₁-C₆-Alkyl, C₁-C₆-Alkyl, C₁-C₆-Heteroalkyl, Halogen, CN, C₃-C₁₂-Cycloalkyl, C₃-C₁₂-Heterocyclyl substituiert sind, ausgewählt ist;
wobei R¹² aus H, NH₂, NHC₁-C₆-Alkyl, C₁-C₆-Alkyl, C₁-C₆-Heteroalkyl, C₂-C₆-Alkenyl, C₂-C₆-Alkinyl, C₃-C₁₂-Cycloalkyl und C₃-C₁₂-Heterocyclyl ausgewählt ist; und gegebenenfalls ein oder mehrere pharmazeutisch unbedenkliche Verdünnungsmittel, Hilfsstoffe oder Träger; mit der Maßgabe, dass, falls R² C₁-C₆-Alkyl oder C₃-C₁₂-Cycloalkyl ist, Y¹ N ist.

2. T-Zell-Modulator (TCM) zur Verwendung nach Anspruch 1, wobei der T-Zell-Modulator (TCM) aus der Gruppe bestehend aus 6-((4'-Fluor-[1,1'-biphenyl]-4-yl)oxy)-2-methylpyridin-3-amin, N-Methyl-6-(4-thiazol-5-yl)phenoxy)pyridin-3-amin und 6-(4-(*tert*-Butylphenoxy)pyridin-3-amin ausgewählt ist, oder pharmazeutisch unbedenkliche Salze, Hydrate, Solvate oder Stereoisomere davon.

3. T-Zell-Modulator (TCM) zur Verwendung nach Anspruch 1, wobei es sich bei dem T-Zell-Modulator (TCM) um 6-(4-(*tert*-Butylphenoxy)pyridin-3-amin handelt, oder pharmazeutisch unbedenkliche Salze, Hydrate, Solvate oder Stereoisomere davon.

4. T-Zell-Modulator (TCM) zur Verwendung nach einem der Ansprüche 1-3, wobei die Autoimmun- und Entzündungskrankheit ausgewählt ist aus der Gruppe bestehend aus akuter, chronischer und steroidrefraktärer Graft-versus-Host-Krankheit (GvHD), Typ-1-Diabetes, entzündlicher Darmerkrankung, rheumatoider Arthritis, Sjögren-Syndrom, Asthma, Allergien, atopischer Dermatitis, Psoriasis, Entzündung bei Organtransplantation, Abstoßung von Organtransplantaten, Morbus Crohn, Colitis ulcerosa, Psoriasis-Arthritis, systemischem Lupus erythematodes/Lupus nephritis, multipler Sklerose, Neuromyelitis optica, Pemphigus vulgaris, chronischer spontaner Urtikaria, Myasthenia gravis, Uveitis, Autoimmunhepatitis, primärer biliärer Zirrhose, Morbus Behcet, Kawasaki-Krankheit, Vaskulitis, Felty-Syndrom, Guillain-Barre-Syndrom, polyendokrinem Autoimmunsyndrom 1, polyendokrinem Syndrom Typ 2, Autoimmunpankreatitis, Zöliakie, Morbus Addison und Autoimmunthyreoiditis.

5. T-Zell-Modulator (TCM) zur Verwendung nach einem der Ansprüche 1-3, wobei die Autoimmun- und Entzündungskrankheit durch Th1-Zellen getrieben wird und aus der Gruppe bestehend aus akuter GvHD, chronischer GvHD, Typ-1-Diabetes, entzündlicher Darmerkrankung, Morbus Crohn, Ulceritis, rheumatoider Arthritis, Psoriasis, Psoriasis-Arthritis, multipler Sklerose, Sjögren-Syndrom ausgewählt ist.

6. T-Zell-Modulator (TCM) zur Verwendung nach einem der Ansprüche 1-3, wobei die Autoimmun- und Entzündungskrankheit aus der Gruppe bestehend aus atopischer Dermatitis und Asthma ausgewählt ist oder wobei die Autoimmun- und Entzündungskrankheit durch Th17-Zellen getrieben wird und es sich dabei um Psoriasis handelt.

7. T-Zell-Modulator (TCM) zur Verwendung nach einem der Ansprüche 1-3, wobei die Autoimmun- und Entzündungskrankheit durch Zellen des angeborenen Immunsystems getrieben wird, die aus der Gruppe bestehend aus Makrophagen und dendritischen Zellen ausgewählt ist.

8. T-Zell-Modulator (TCM) zur Verwendung nach einem der Ansprüche 1-3, wobei es sich bei der Autoimmun- und Entzündungskrankheit um Graft-versus-Host-Krankheit (GvHD) handelt.

9. T-Zell-Modulator (TCM) zur Verwendung nach einem der Ansprüche 1-3, wobei es sich bei der Autoimmun- und Entzündungskrankheit um akute, chronische und steroidrefraktäre Graft-versus-Host-Krankheit (GvHD) handelt.

10. Pharmazeutische Zusammensetzung zur Verwendung bei einem Verfahren zur Vorbeugung, Verzögerung des Fortschreitens oder Behandlung einer Autoimmun- und Entzündungskrankheit, umfassend den T-Zell-Modulator (TCM) nach einem der Ansprüche 1-3 und gegebenenfalls ein oder mehrere pharmazeutisch unbedenkliche Verdünnungsmittel, Hilfsstoffe oder Träger.

11. Kit zur Verwendung bei einem Verfahren zur Vorbeugung, Verzögerung des Fortschreitens oder Behandlung einer Autoimmun- und Entzündungskrankheit bei einem Individuum, umfassend einen Behälter und eine Packungsbeilage, wobei der Behälter mindestens eine Dosis eines Medikaments umfasst, das den T-Zell-Modulator (TCM) nach einem der Ansprüche 1-3 oder die pharmazeutische Zusammensetzung nach Anspruch 10 umfasst, und die Packungsbeilage gegebenenfalls Anweisungen zur Behandlung eines Individuums gegen eine Autoimmun- und Entzündungskrankheit (AIID) unter Verwendung des Medikaments umfasst.

## Revendications

1. Modulateur des lymphocytes T (TCM) destiné à être utilisé dans une méthode de prévention, de ralentissement de la progression ou de traitement d'une maladie auto-immune et inflammatoire (AIID) chez un sujet, le modulateur des lymphocytes T (TCM) étant un inhibiteur du complexe de transcription NOTCH et le modulateur des lymphocytes T (TCM) étant un composé de formule (I) 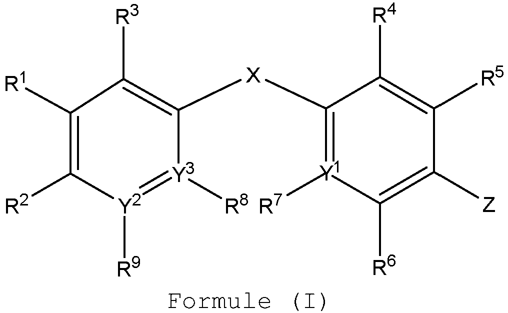
sels pharmaceutiquement acceptables, hydrates, solvates ou stéréoisomères de celui-ci,
X étant choisi parmi CH₂, CF₂, CHF, CO, CHOH, CHO-alkyle en C₁-C₃, NH, N(alkyle en C₁-C₃), S, SO et O ;
Y¹, Y², et Y³ étant chacun indépendamment choisis entre N et C ;
Z étant NR¹⁰R¹¹, R¹⁰ et R¹¹ étant chacun indépendamment choisis entre H et alkyle en C₁-C₆ ;
R¹ étant choisi parmi H, halogène, alkyle en C₁-C₆, cycloalkyle en C₃-C₁₂, hétérocyclyle en C₃-C₁₂, alkoxy en C₁-C₆, hétéroalkyle en C₁-C₆, (alkyl en C₀-C₃)-O-(alkyl en C₀-C₃)aryle, l'aryle étant éventuellement substitué par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ; (alkyl en C₀-C₃)-O-(alkoxyalkyl en C₀-C₃)hétéroaryle, l'hétéroaryle étant éventuellement substitué par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ; et alkyle en C₁-C₆ substitué par aryle ou hétéroaryle, l'aryle et l'hétéroaryle étant éventuellement substitués par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ;
R² étant choisi parmi alkyle en C₁-C₆, cycloalkyle en C₃-C₁₂, aryle et hétéroaryle, l'aryle et l'hétéroaryle étant éventuellement substitués par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂, C(O)R¹², (alkyl en C₁-C₆)-C(O)R¹² ;
R³ étant choisi parmi H, halogène, alkyle en C₁-C₆, hétéroalkyle en C₁-C₆, cycloalkyle en C₃-C₁₂, hétérocyclyle en C₃-C₁₂ et alkoxy en C₁-C₆ ;
R⁴, R⁵ et R⁶ étant chacun indépendamment choisis parmi H, halogène, CN, alkoxy en C₁-C₆, S-alkyle en C₁-C₆, alkyle en C₁-C₆, hétéroalkyle en C₁-C₆, cycloalkyle en C₃-C₁₂ et hétérocyclyle en C₃-C₁₂ ;
R⁷ étant absent lorsque Y¹ est N ou étant choisi parmi H, halogène, alkyle en C₁-C₆, hétéroalkyle en C₁-C₆, cycloalkyle en C₃-C₁₂, hétérocyclyle en C₃-C₁₂ et alkoxy en C₁-C₆ lorsque Y¹ est C ;
R⁸ étant absent lorsque Y³ est N ou étant choisi parmi H, halogène, alkyle en C₁-C₆, hétéroalkyle en C₁-C₆, cycloalkyle en C₃-C₁₂, hétérocyclyle en C₃-C₁₂ et alkoxy en C₁-C₆ lorsque Y³ est C ;
R⁹ étant absent lorsque Y² est N ou étant choisi parmi H, halogène, alkyle en C₁-C₆, cycloalkyle en C₃-C₁₂, hétérocyclyle en C₃-C₁₂, alkoxy en C₁-C₆, hétéroalkyle en C₁-C₆, (alkyl en C₀-C₃)-O-(alkyl en C₀-C₃)aryle, l'aryle étant éventuellement substitué par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ; (alkyl en C₀-C₃)-O-(alkyl en C₀-C₃)hétéroaryle, l'hétéroaryle étant éventuellement substitué par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ; et alkyle en C₁-C₆ substitué par aryle ou hétéroaryle, l'aryle et l'hétéroaryle étant éventuellement substitués par NH₂, O-alkyle en C₁-C₆, alkyle en C₁-C₆, hétéroalkyle en C₁-C₆, halogène, CN, cycloalkyle en C₃-C₁₂, hétérocyclyle en C₃-C₁₂ ;
R¹² étant choisi parmi H, NH₂, NH-alkyle en C₁-C₆, alkyle en C₁-C₆, hétéroalkyle en C₁-C₆, alcényle en C₂-C₆, alcynyle en C₂-C₆, cycloalkyle en C₃-C₁₂ et hétérocyclyle en C₃-C₁₂ ; et éventuellement un ou plusieurs diluants, excipients ou véhicules pharmaceutiquement acceptables ; à condition que si R² est alkyle en C₁-C₆ ou cycloalkyle en C₃-C₁₂, Y¹ soit N.

2. Modulateur des lymphocytes T (TCM) destiné à être utilisé selon la revendication 1, le modulateur des lymphocytes T (TCM) étant choisi dans le groupe constitué par la 6-((4'-fluoro-[1,1'-biphényl]-4-yl)oxy)-2-méthylpyridin-3-amine, la N-méthyl-6-(4-(thiazol-5-yl)phénoxy)pyridin-3-amine et la 6-(4-tert-butylphénoxy)pyridin-3-amine ou les sels pharmaceutiquement acceptables, hydrates, solvates ou stéréoisomères de celles-ci.

3. Modulateur des lymphocytes T (TCM) destiné à être utilisé selon la revendication 1, le modulateur des lymphocytes T (TCM) étant la 6-(4-(tert-butylphénoxy)pyridin-3-amine ou des sels pharmaceutiquement acceptables, hydrates, solvates ou stéréoisomères de celle-ci.

4. Modulateur des lymphocytes T (TCM) destiné à être utilisé selon l'une quelconque des revendications 1 à 3, la maladie auto-immune et inflammatoire étant choisie dans le groupe constitué par la maladie du greffon contre l'hôte (GvHD) aiguë, chronique et réfractaire aux stéroïdes, le diabète de type 1, une maladie intestinale inflammatoire, la polyarthrite rhumatoïde, le syndrome de Sjögren, l'asthme, les allergies, une dermatite atopique, le psoriasis, une inflammation en transplantation d'organe, le rejet de transplant d'organe, la maladie de Crohn, la recto-colite hémorragique, le rhumatisme psoriasique, le lupus érythémateux disséminé/la néphrite lupique, la sclérose en plaques, la neuromyélite optique, le pemphigus vulgaire, l'urticaire chronique spontanée, la myasthénie grave, une uvéite, une hépatite auto-immune, la cirrhose biliaire primitive, la maladie de Behçet, la maladie de Kawasaki, une vascularite, le syndrome de Felty, le syndrome de Guillain-Barré, le syndrome polyglandulaire auto-immun de type 1, le syndrome polyglandulaire de type 2, une pancréatite auto-immune, la maladie cœliaque, la maladie d'Addison et une thyroïdite auto-immune.

5. Modulateur des lymphocytes T (TCM) destiné à être utilisé selon l'une quelconque des revendications 1 à 3, la maladie auto-immune et inflammatoire étant médiée par les cellules Th1 et étant choisie dans le groupe constitué par la GvHD aiguë, la GvHD chronique, le diabète de type 1, une maladie intestinale inflammatoire, la maladie de Crohn, une ulcérite, la polyarthrite rhumatoïde, le psoriasis, le rhumatisme psoriasique, la sclérose en plaques, le syndrome de Sjögren.

6. Modulateur des lymphocytes T (TCM) destiné à être utilisé selon l'une quelconque des revendications 1 à 3, la maladie auto-immune et inflammatoire étant choisie dans le groupe constitué par une dermatite atopique et l'asthme ou la maladie auto-immune et inflammatoire étant médiée par les cellules Th17 et étant le psoriasis.

7. Modulateur des lymphocytes T (TCM) destiné à être utilisé selon l'une quelconque des revendications 1 à 3, la maladie auto-immune et inflammatoire étant médiée par des cellules du système immunitaire inné choisies dans le groupe constitué par les macrophages et les cellules dendritiques.

8. Modulateur des lymphocytes T (TCM) destiné à être utilisé selon l'une quelconque des revendications 1 à 3, la maladie auto-immune et inflammatoire étant la maladie du greffon contre l'hôte (GvHD).

9. Modulateur des lymphocytes T (TCM) destiné à être utilisé selon l'une quelconque des revendications 1 à 3, la maladie auto-immune et inflammatoire étant la maladie du greffon contre l'hôte (GvHD) aiguë, chronique et réfractaire aux stéroïdes.

10. Composition pharmaceutique destinée à être utilisée dans une méthode de prévention, de ralentissement de la progression ou de traitement d'une maladie auto-immune et inflammatoire comprenant le modulateur des lymphocytes T (TCM) selon l'une quelconque des revendications 1 à 3 et éventuellement un ou plusieurs diluants, excipients ou véhicules pharmaceutiquement acceptables.

11. Kit destiné à être utilisé dans une méthode de prévention, de ralentissement de la progression ou de traitement d'une maladie auto-immune et inflammatoire chez un sujet, comprenant un récipient et une notice, le récipient comprenant au moins une dose d'un médicament comprenant le modulateur des lymphocytes T (TCM) selon l'une quelconque des revendications 1 à 3 ou la composition pharmaceutique selon la revendication 10 et la notice comprenant éventuellement des instructions pour le traitement d'un sujet pour une maladie auto-immune et inflammatoire (AIID) à l'aide du médicament.
